# EUROPEAN PATENT APPLICATION

(11) **EP 2 239 323 A1**
(43) Date of publication of application: **13.10.2010**
(21) Application number: 09700866.8
(22) Date of filing: 09.01.2009
(51) Int. Cl.: C12N 15/00, A61K 39/395, A61P 29/00, C07K 16/28, C07K 16/46, C12N 1/15, C12N 1/19, C12N 1/21, C12N 5/10, C12N 15/09, C12P 21/08

(54) **HUMANIZED ANTI-HUMAN 9-INTEGRIN ANTIBODY**

(30) Priority: 11.01.2008 JP 2008004975; 31.10.2008 JP 2008282496
(71) Applicant: Juridical Foundation The Chemo-Sero-Therapeutic Research Institute, Kumamoto-shi Kumamoto 860-8568 (JP); Astellas Pharma Inc., Tokyo 103-8411 (JP)
(72) Inventor: UEHARA, Kenji, Kikuchi-shi Kumamoto 869-1298 (JP); HIGUCHI, Hirofumi, Kikuchi-shi Kumamoto 869-1298 (JP); NAKASHIMA, Toshihiro, Kikuchi-shi Kumamoto 869-1298 (JP); ISHIKAWA, Daisuke, Kikuchi-shi Kumamoto 869-1298 (JP); YAMAMOTO, Nobuchika, Tokyo 103-8411 (JP); FUJITA, Hirotada, Tokyo 103-8411 (JP); SAKAI, Fumihiko, Tokyo 103-8411 (JP)
(74) Representative: Gille Hrabal Struck Neidlein Prop Roos
(86) International application number: PCT/JP2009/050188
(87) International publication number: WO 2009/088065

(57) **Abstract**

The present invention provides a humanized anti-human α9 integrin antibody having improved activity and/or property as compared to donor mouse anti-human α9 integrin antibody, namely, a humanized anti-human α9 integrin antibody comprising a heavy-chain variable region consisting of the amino acid sequence shown by SEQ ID NO: 1 or the amino acid sequence shown by SEQ ID NO: 1 wherein one or several amino acids are substituted, deleted, inserted and/or added and a light-chain variable region consisting of the amino acid sequence shown by SEQ ID NO: 61 or the amino acid sequence shown by SEQ ID NO: 61 wherein one or several amino acids are substituted, deleted, inserted and/or added, as well as a means for preventing or treating various diseases involving human α9 integrin in their pathogenesis, which uses the antibody.

SEQUENCE LISTING

<110>  Juridical Foundation The Chemo-Sero-Therapeutic Research Institute
       Astellas Pharma Inc.

<120>  Humanized anti - human alpha9 integrin antibody

<130>  091326

<150>  JP 2008-004975
<151>  2008-01-11

<150>  JP 2008-282496
<151>  2008-10-31

<160>  157

<170>  PatentIn version 3.4

<210>  1
<211>  120
<212>  PRT
<213>  Artificial

<220>
<223>  VH chain of humanized anti-human alpha9 integrin antibody

<400>  1

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  2
<211>  120
<212>  PRT
<213>  Artificial

<220>
<223>  VH chain of humanized anti-human alpha9 integrin antibody

<400>  2

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210> 3
<211> 120
<212> PRT
<213> Artificial

<220>
<223> VH chain of humanized anti-human alpha9 integrin antibody

<400> 3

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210> 4
<211> 120
<212> PRT
<213> Artificial

<220>
<223>  VH chain of humanized anti-human alpha9 integrin antibody

<400>  4

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  5
<211>  120
<212>  PRT
<213>  Artificial

<220>
<223>  VH chain of humanized anti-human alpha9 integrin antibody

<400>  5

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120


<210>  6
<211>  120
<212>  PRT
<213>  Artificial

<220>
<223>  VH chain of humanized anti-human alpha9 integrin antibody

<400>  6

Gln Val Gln Leu Val Gln Ser Gly Ala Glu Val Lys Lys Pro Gly Ser
1               5                   10                  15


Ser Val Lys Met Ser Cys Lys Ala Ser Gly Tyr Thr Leu Thr Thr Tyr
            20                  25                  30


Trp Ile His Trp Val Lys Gln Arg Pro Gly Gln Gly Leu Glu Trp Ile
        35                  40                  45


Gly Tyr Ile Asn Pro Ser Ser Gly Tyr Thr Glu Tyr Asn Gln Lys Phe
    50                  55                  60


Lys Asp Arg Val Thr Ile Thr Ala Asp Lys Ser Thr Ser Thr Ala Tyr
65                  70                  75                  80


Met Glu Leu Ser Ser Leu Arg Ser Glu Asp Thr Ala Val Tyr Tyr Cys
                85                  90                  95


Ala Ile Tyr Gly Asp Tyr Gly Asp Phe Tyr Phe Asp Tyr Trp Gly Gln
            100                 105                 110


Gly Thr Thr Val Thr Val Ser Ser
        115                 120


<210>  7
<211>  120
<212>  PRT
<213>  Artificial

<220>
<223>  VH chain of humanized anti-human alpha9 integrin antibody

<400>  7

Gln Val Gln Leu Val Gln Ser Gly Ala Glu Val Lys Lys Pro Gly Ser
1               5                   10                  15


Ser Val Lys Met Ser Cys Lys Ala Ser Gly Tyr Thr Leu Thr Thr Tyr
            20                  25                  30


Trp Ile His Trp Val Lys Gln Arg Pro Gly Gln Gly Leu Glu Trp Ile
        35                  40                  45


Gly Tyr Ile Asn Pro Ser Ser Gly Tyr Thr Glu Tyr Asn Gln Lys Phe
    50                  55                  60


Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210>  8
<211>  120
<212>  PRT
<213>  Artificial

<220>
<223>  VH chain of humanized anti-human alpha9 integrin antibody

<400>  8

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  9
<211>  120
<212>  PRT
<213>  Artificial

<220>
<223>  VH chain of humanized anti-human alpha9 integrin antibody

<400>  9

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  10
<211>  120
<212>  PRT
<213>  Artificial

<220>
<223>  VH chain of humanized anti-human alpha9 integrin antibody

<400>  10

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  11
<211>  120
<212>  PRT
<213>  Artificial

<220>
<223>  VH chain of humanized anti-human alpha9 integrin antibody

<400>  11

Gln Val Gln Leu Val Gln Ser Gly Ala Glu Val Lys Lys Pro Gly Ser

1                   5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  12
<211>  120
<212>  PRT
<213>  Artificial

<220>
<223>  VH chain of humanized anti-human alpha9 integrin antibody

<400>  12

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210>  13
<211>  120

<212>  PRT
<213>  Artificial

<220>
<223>  VH chain of humanized anti-human alpha9 integrin antibody

<400>  13

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  14
<211>  120
<212>  PRT
<213>  Artificial

<220>
<223>  VH chain of humanized anti-alpha9 integrin antibody

<400>  14

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln

            100                 105                 110

Gly Thr Thr Val Thr Val Ser Ser
        115                 120

<210> 15
<211> 120
<212> PRT
<213> Artificial

<220>
<223> VH chain of humanized anti-human alpha9 integrin antibody

<400> 15

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210> 16
<211> 120
<212> PRT
<213> Artificial

<220>
<223> VH chain of humanized anti-human alpha9 integrin antibody

<400> 16

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  17
<211>  120
<212>  PRT
<213>  Artificial

<220>
<223>  VH chain of humanized anti-human alpha9 integrin antibody

<400>  17

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210>  18
<211>  120
<212>  PRT
<213>  Artificial

<220>
<223>  VH chain of humanized anti-human alpha9 integrin antibody

<400>  18

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  19
<211>  120
<212>  PRT
<213>  Artificial

<220>
<223>  VH chain of humanized anti-human alpha9 integrin antibody

<400>  19

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  20
<211>  120
<212>  PRT
<213>  Artificial

<220>
<223>  VH chain of humanized anti-human alpha9 integrin antibody

<400> 20

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  21
<211>  120
<212>  PRT
<213>  Artificial

<220>
<223>  VH chain of humanized anti-human alpha9 integrin antibody

<400>  21

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  22
<211>  120
<212>  PRT
<213>  Artificial

<220>
<223>  VH chain of humanized anti-human alpha9 integrin antibody

<400>  22

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120


<210>  23
<211>  120
<212>  PRT
<213>  Artificial

<220>
<223>  VH chain of humanized anti-human alpha9 integrin antibody

<400>  23

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  24
<211>  120
<212>  PRT
<213>  Artificial

<220>
<223>  VH chain of humanized anti-human alpha9 integrin antibody

<400>  24

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  25
<211>  120
<212>  PRT
<213>  Artificial

<220>
<223>  VH chain of humanized anti-human alpha9 integrin antibody

<400>  25

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe

50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  26
<211>  120
<212>  PRT
<213>  Artificial

<220>
<223>  VH chain of humanized anti-human alpha9 integrin antibody

<400>  26

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  27
<211>  120
<212>  PRT
<213>  Artificial

<220>
<223>  VH chain of humanized anti-human alpha9 integrin antibody

<400>  27

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210> 28
<211> 120
<212> PRT
<213> Artificial

<220>
<223> VH chain of humanized anti-human alpha9 integrin antibody

<400> 28

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210> 29
<211> 120
<212> PRT
<213> Artificial

<220>
<223>  VH chain of humanized anti-human alpha9 integrin antibody

<400>  29

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  30
<211>  120
<212>  PRT
<213>  Artificial

<220>
<223>  VH chain of humanized anti-human alpha9 integrin antibody

<400>  30

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  31
<211>  120
<212>  PRT
<213>  Artificial

<220>
<223>  VH chain of humanized anti-human alpha9 integrin antibody

<400>  31

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  32
<211>  120
<212>  PRT
<213>  Artificial

<220>
<223>  VH chain of humanized anti-human alpha9 integrin antibody

<400>  32

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210> 33
<211> 120
<212> PRT
<213> Artificial

<220>
<223> VH chain of humanized anti-human alpha9 integrin antibody

<400> 33

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210> 34
<211> 120
<212> PRT
<213> Artificial

<220>
<223> VH chain of humanized anti-human alpha9 integrin antibody

<400> 34

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  35
<211>  120
<212>  PRT
<213>  Artificial

<220>
<223>  VH chain of humanized anti-human alpha9 integrin antibody

<400>  35

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  36
<211>  120
<212>  PRT
<213>  Artificial

<220>
<223>  VH chain of humanized anti-human alpha9 integrin antibody

<400>  36

Gln Val Gln Leu Val Gln Ser Gly Ala Glu Val Lys Lys Pro Gly Ser

1                   5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  37
<211>  120
<212>  PRT
<213>  Artificial

<220>
<223>  VH chain of humanized anti-human alpha9 integrin antibody

<400>  37

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210>  38
<211>  120

<212>  PRT
<213>  Artificial

<220>
<223>  VH chian of humanized anti-human alpha9 integrin antibody

<400>  38

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  39
<211>  120
<212>  PRT
<213>  Artificial

<220>
<223>  VH chain of humanized anti-human alpha9 integrin antibody

<400>  39

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln

            100                 105                 110

Gly Thr Thr Val Thr Val Ser Ser
        115                 120

<210> 40
<211> 120
<212> PRT
<213> Artificial

<220>
<223> VH chain of humanized anti-human alpha9 integrin antibody

<400> 40

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210> 41
<211> 120
<212> PRT
<213> Artificial

<220>
<223> VH chain of humanized anti-human alpha9 integrin antibody

<400> 41

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  42
<211>  120
<212>  PRT
<213>  Artificial

<220>
<223>  VH chain of humanized anti-human alpha9 integrin antibody

<400>  42

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210>  43
<211>  120
<212>  PRT
<213>  Artificial

<220>
<223>  VH chain of humanized anti-human alpha9 integrin antibody

<400>  43

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  44
<211>  120
<212>  PRT
<213>  Artificial

<220>
<223>  VH chain of humanized anti-human alpha9 integrin antibody

<400>  44

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  45
<211>  120
<212>  PRT
<213>  Artificial

<220>
<223>  VH chain of humanized anti-human alpha9 integrin antibody

<400> 45

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  46
<211>  120
<212>  PRT
<213>  Artificial

<220>
<223>  VH chain of humanized anti-human alpha9 integrin antibody

<400>  46

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210> 47
<211> 120
<212> PRT
<213> Artificial

<220>
<223> VH chain of humanized anti-human alpha9 integrin antibody

<400> 47

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120


<210> 48
<211> 120
<212> PRT
<213> Artificial

<220>
<223> VH chain of humanized anti-human alpha9 integrin antibody

<400> 48

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  49
<211>  120
<212>  PRT
<213>  Artificial

<220>
<223>  VH chain of humanized anti-human alpha9 integrin antibody

<400>  49

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  50
<211>  120
<212>  PRT
<213>  Artificial

<220>
<223>  VH chain of humanized anti-human alpha9 integrin antibody

<400>  50

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe

50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  51
<211>  120
<212>  PRT
<213>  Artificial

<220>
<223>  VH chain of humanized anti-human alpha9 integrin antibody

<400>  51

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  52
<211>  120
<212>  PRT
<213>  Artificial

<220>
<223>  VH chain of humanized anti-human alpha9 integrin antibody

<400>  52

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210> 53
<211> 120
<212> PRT
<213> Artificial

<220>
<223> VH chain of humanized anti-human alpha9 integrin antibody

<400> 53

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210> 54
<211> 120
<212> PRT
<213> Artificial

<220>
<223>  VH chain of humanized anti-human alpha9 integrin antibody

<400>  54

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120


<210>  55
<211>  120
<212>  PRT
<213>  Artificial

<220>
<223>  VH chain of humanized anti-human alpha9 integrin antibody

<400>  55

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Ile His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  56
<211>  120
<212>  PRT
<213>  Artificial

<220>
<223>  VH chain of humanized anti-human alpha9 integrin antibody

<400>  56

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser
        115                 120

<210>  57
<211>  120
<212>  PRT
<213>  Artificial

<220>
<223>  VH chain of humanized anti-human alpha9 integrin antibody

<400>  57

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Leu Tyr Phe Asp Tyr Trp Gly Gln
            100                 105                 110

Gly Thr Thr Val Thr Val Ser Ser
        115                 120

<210>  58
<211>  120
<212>  PRT
<213>  Artificial

<220>
<223>  VH chain of humanized anti-human alpha9 integrin antibody

<400>  58

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser
        115                 120

<210>  59
<211>  120
<212>  PRT
<213>  Artificial

<220>
<223>  VH chain of humanized anti-human alpha9 integrin antibody

<400>  59

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ser Trp Tyr Phe Asp Tyr Trp Gly Gln
            100                 105                 110

Gly Thr Thr Val Thr Val Ser Ser
        115                 120

<210>  60
<211>  120
<212>  PRT
<213>  Artificial

<220>
<223>  VH chain of humanized anti-human alpha9 integrin antibody

<400>  60

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Trp Glu Tyr Phe Asp Tyr Trp Gly Gln
            100                 105                 110

Gly Thr Thr Val Thr Val Ser Ser
        115                 120

<210>  61
<211>  108
<212>  PRT
<213>  Artificial

<220>
<223>  VL chain of humanized anti-human alpha9 integrin antibody

<400>  61

Asp Ile Gln Met Thr Gln Ser Pro Ser Ser Leu Ser Ala Ser Val Gly

1               5                   10                  15

Asp Arg Val Thr Ile Thr Cys Lys Ala Ser Gln Ser Val Asn Thr Asp
            20                  25                  30

Val Ala Trp Tyr Gln Gln Lys Pro Gly Lys Ala Pro Lys Leu Leu Ile
        35                  40                  45

Tyr Phe Ala Ser Asn His Tyr Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Arg Gln Asp Tyr Ser Ser Pro Phe
                85                  90                  95

Thr Phe Gly Gly Gly Thr Lys Val Glu Ile Lys Arg
            100                 105

<210>  62
<211>  108
<212>  PRT
<213>  Artificial

<220>
<223>  VL chain of humanized anti-human alpha9 integrin antibody

<400>  62

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Val Asn Thr Asp
            20                  25                  30

Val Ala Trp Tyr Gln Gln Lys Pro Gly Lys Ala Pro Lys Leu Leu Ile
        35                  40                  45

Tyr Phe Ala Ser Asn His Tyr Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Arg Gln Asp Tyr Ser Ser Pro Phe
                85                  90                  95

Thr Phe Gly Gly Gly Thr Lys Val Glu Ile Lys Arg
            100                 105

<210>  63
<211>  108
<212>  PRT
<213>  Artificial

<220>
<223>  VL chain of huminzed anti-human alpha9 integrin antibody

<400>  63

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Asn Thr Asp
            20                  25                  30

Val Ala Trp Tyr Gln Gln Lys Pro Gly Lys Ala Pro Lys Leu Leu Ile
        35                  40                  45

Tyr Phe Ala Ser Asn His Tyr Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Arg Gln Asp Tyr Ser Ser Pro Phe
                85                  90                  95

Thr Phe Gly Gly Gly Thr Lys Val Glu Ile Lys Arg
            100                 105

<210>  64
<211>  360
<212>  DNA
<213>  Artificial

<220>
<223>  VH gene of humanized anti-human alpha9 integrin antibody

<400>  64
caggtgcagc tggtgcagtc tggggctgag gtgaagaagc ctgggtcctc cgtgaagatg      60

tcctgcaagg cttctggata caccctcacc acctactgga tgcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg atttctactt tgactactgg ggccaaggga ccacggtcac cgtctcctca     360

<210>  65
<211>  360
<212>  DNA
<213>  Artificial

<220>
<223>  VH gene of humanized anti-human alpha9 integrin antibody

<400>  65
caggtgcagc tggtgcagtc tggggctgag gtgaagaagc ctgggtcctc cgtgaagatg      60

tcctgcaagg cttctggata caccctcacc acctactgga tgcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg ctttgtactt tgactactgg ggccaaggga ccacggtcac cgtctcctca     360

<210>  66
<211>  360
<212>  DNA

<213>  Artificial

<220>
<223>  VH gene of humanized anti-human alpha9 integrin antibody

<400>  66
caggtgcagc tggtgcagtc tggggctgag gtgaagaagc ctgggtcctc cgtgaagatg      60

tcctgcaagg cttctggata caccctcacc acctactgga tgcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg cttggtactt tgactactgg gggcaaggga ccacggtcac cgtctcctca     360

<210>  67
<211>  360
<212>  DNA
<213>  Artificial

<220>
<223>  VH gene of humanized anti-human alpha9 integrin antibody

<400>  67
caggtgcagc tggtgcagtc tggggctgag gtgaagaagc ctgggtcctc cgtgaagatg      60

tcctgcaagg cttctggata caccctcacc acctactgga tgcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggt cttggtactt tgactactgg gggcaaggga ccacggtcac cgtctcctca     360

<210>  68
<211>  360
<212>  DNA
<213>  Artificial

<220>
<223>  VH gene of humanized anti-human alpha9 integrin antibody

<400>  68
caggtgcagc tggtgcagtc tggggctgag gtgaagaagc ctgggtcctc cgtgaagatg      60

tcctgcaagg cttctggata caccctcacc acctactgga tgcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggt gggaatactt tgactactgg gggcaaggga ccacggtcac cgtctcctca     360

<210>  69
<211>  360
<212>  DNA
<213>  Artificial

<220>
<223>  VH gene of humanized anti-human alpha9 integrin antibody

<400>  69
caggtgcagc tggtgcagtc tggggctgag gtgaagaagc ctgggtcctc cgtgaagatg      60

tcctgcaagg cttctggata caccctcacc acctactgga ttcactgggt gaaacagaga     120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg atttctactt tgactactgg gggcaaggga ccacggtcac cgtctcctca 360

<210> 70
<211> 360
<212> DNA
<213> Artificial

<220>
<223> VH gene of humanized anti-human alpha9 integrin antibody

<400> 70
caggtgcagc tggtgcagtc tggggctgag gtgaagaagc ctgggtcctc cgtgaagatg 60

tcctgcaagg cttctggata caccctcacc acctactgga ttcactgggt gaaacagaga 120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg ctttgtactt tgactactgg gggcaaggga ccacggtcac cgtctcctca 360

<210> 71
<211> 360
<212> DNA
<213> Artificial

<220>
<223> VH gene of humanized anti-human alpha9 integrin antibody

<400> 71
caggtgcagc tggtgcagtc tggggctgag gtgaagaagc ctgggtcctc cgtgaagatg 60

tcctgcaagg cttctggata caccctcacc acctactgga ttcactgggt gaaacagaga 120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg cttggtactt tgactactgg gggcaaggga ccacggtcac cgtctcctca 360

<210> 72
<211> 360
<212> DNA
<213> Artificial

<220>
<223> VH gene of humanized anti-human alpha9 integrin antibody

<400> 72
caggtgcagc tggtgcagtc tggggctgag gtgaagaagc ctgggtcctc cgtgaagatg 60

tcctgcaagg cttctggata caccctcacc acctactgga ttcactgggt gaaacagaga 120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggt cttggtactt tgactactgg gggcaaggga ccacggtcac cgtctcctca 360

<210>  73
<211>  360
<212>  DNA
<213>  Artificial

<220>
<223>  VH gene of humanized anti-human alpha9 integrin antibody

<400>  73
caggtgcagc tggtgcagtc tggggctgag gtgaagaagc ctgggtcctc cgtgaagatg      60

tcctgcaagg cttctggata caccctcacc acctactgga ttcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggt gggaatactt tgactactgg ggcaagggga ccacggtcac cgtctcctca     360

<210>  74
<211>  360
<212>  DNA
<213>  Artificial

<220>
<223>  VH gene of humanized anti-human alpha9 integrin antibody

<400>  74
caggtgcagc tggtgcagtc tggggctgag gtgaagaagc ctgggtcctc cgtgaagatg      60

tcctgcaagg cttctggata caccctcacc acctactggt tgcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg atttctactt tgactactgg ggcaagggga ccacggtcac cgtctcctca     360

<210>  75
<211>  360
<212>  DNA
<213>  Artificial

<220>
<223>  VH gene of humanized anti-human alpha9 integrin antibody

<400>  75
caggtgcagc tggtgcagtc tggggctgag gtgaagaagc ctgggtcctc cgtgaagatg      60

tcctgcaagg cttctggata caccctcacc acctactggt tgcactgggt gaaacagaga     120

cctggacaag ggcttgagtg gattggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg ctttgtactt tgactactgg ggcaagggga ccacggtcac cgtctcctca     360

<210>  76
<211>  360
<212>  DNA
<213>  Artificial

<220>
<223>  VH gene of humanized anti-human alpha9 integrin antibody

<400> 76
caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg cttggtactt tgactactgg gggcaaggga ccacggtcac cgtctcctca 360

<210> 77
<211> 360
<212> DNA
<213> Artificial

<220>
<223> VH gene of humanized anti-human alpha9 integrin antibody

<400> 77
caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggt cttggtactt tgactactgg gggcaaggga ccacggtcac cgtctcctca 360

<210> 78
<211> 360
<212> DNA
<213> Artificial

<220>
<223> VH gene of humanized anti-human alpha9 integrin antibody

<400> 78
caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggt gggaatactt tgactactgg gggcaaggga ccacggtcac cgtctcctca 360

<210> 79
<211> 360
<212> DNA
<213> Artificial

<220>
<223> VH gene of humanized anti-human alpha9 integrin antibody

<400> 79
caggtgcagc tggtgcagtc tggggctgag gtgaagaagc ctgggtcctc cgtgaagatg 60

tcctgcaagg cttctggata caccctcacc acctactgga tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg atttctactt tgactactgg gggcaaggga ccacggtcac cgtctcctca 360

<210> 80
<211> 360
<212> DNA
<213> Artificial

<220>
<223> VH gene of humanized anti-human alpha9 integrin antibody

<400> 80
caggtgcagc tggtgcagtc tggggctgag gtgaagaagc ctgggtcctc cgtgaagatg 60

tcctgcaagg cttctggata caccctcacc acctactgga tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg ctttgtactt tgactactgg gggcaaggga ccacggtcac cgtctcctca 360

<210> 81
<211> 360
<212> DNA
<213> Artificial

<220>
<223> VH gene of humanized anti-human alpha9 integrin antibody

<400> 81
caggtgcagc tggtgcagtc tggggctgag gtgaagaagc ctgggtcctc cgtgaagatg 60

tcctgcaagg cttctggata caccctcacc acctactgga tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg cttggtactt tgactactgg gggcaaggga ccacggtcac cgtctcctca 360

<210> 82
<211> 360
<212> DNA
<213> Artificial

<220>
<223> VH gene of humanized anti-human alpha9 integrin antibody

<400> 82
caggtgcagc tggtgcagtc tggggctgag gtgaagaagc ctgggtcctc cgtgaagatg 60

tcctgcaagg cttctggata caccctcacc acctactgga tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggt cttggtactt tgactactgg gggcaaggga ccacggtcac cgtctcctca 360

<210> 83
<211> 360
<212> DNA
<213> Artificial

<220>
<223>  VH gene of humanized anti-human alpha9 integrin antibody

<400>  83
caggtgcagc tggtgcagtc tggggctgag gtgaagaagc ctgggtcctc cgtgaagatg      60

tcctgcaagg cttctggata caccctcacc acctactgga tgcactgggt gaaacagaga     120

cctggacaag ggcttgagtg gattggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggt gggaatactt tgactactgg ggccaaggga ccacggtcac cgtctcctca     360

<210>  84
<211>  360
<212>  DNA
<213>  Artificial

<220>
<223>  VH gene of humanized anti-human alpha9 integrin antibody

<400>  84
caggtgcagc tggtgcagtc tggggctgag gtgaagaagc ctgggtcctc cgtgaagatg      60

tcctgcaagg cttctggata caccctcacc acctactgga ttcactgggt gaaacagaga     120

cctggacaag ggcttgagtg gattggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg atttctactt tgactactgg ggccaaggga ccacggtcac cgtctcctca     360

<210>  85
<211>  360
<212>  DNA
<213>  Artificial

<220>
<223>  VH gene of humanized anti-human alpha9 integrin antibody

<400>  85
caggtgcagc tggtgcagtc tggggctgag gtgaagaagc ctgggtcctc cgtgaagatg      60

tcctgcaagg cttctggata caccctcacc acctactgga ttcactgggt gaaacagaga     120

cctggacaag ggcttgagtg gattggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg ctttgtactt tgactactgg ggccaaggga ccacggtcac cgtctcctca     360

<210>  86
<211>  360
<212>  DNA
<213>  Artificial

<220>
<223>  VH gene of humanized anti-human alpha9 integrin antibody

<400>  86
caggtgcagc tggtgcagtc tggggctgag gtgaagaagc ctgggtcctc cgtgaagatg      60

tcctgcaagg cttctggata caccctcacc acctactgga ttcactgggt gaaacagaga     120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg cttggtactt tgactactgg ggcaagggga ccacggtcac cgtctcctca 360

<210> 87
<211> 360
<212> DNA
<213> Artificial

<220>
<223> VH gene of humanized anti-human alpha9 integrin antibody

<400> 87
caggtgcagc tggtgcagtc tggggctgag gtgaagaagc ctgggtcctc cgtgaagatg 60

tcctgcaagg cttctggata caccctcacc acctactgga ttcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggt cttggtactt tgactactgg ggcaagggga ccacggtcac cgtctcctca 360

<210> 88
<211> 360
<212> DNA
<213> Artificial

<220>
<223> VH gene of humanized anti-human alpha9 integrin antibody

<400> 88
caggtgcagc tggtgcagtc tggggctgag gtgaagaagc ctgggtcctc cgtgaagatg 60

tcctgcaagg cttctggata caccctcacc acctactgga ttcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggt gggaatactt tgactactgg ggcaagggga ccacggtcac cgtctcctca 360

<210> 89
<211> 360
<212> DNA
<213> Artificial

<220>
<223> VH gene of humanized anti-human alpha9 integrin antibody

<400> 89
caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg atttctactt tgactactgg ggcaagggga ccacggtcac cgtctcctca 360

<210> 90
<211> 360
<212> DNA
<213> Artificial

<220>
<223> VH gene of humanized anti-human alpha9 integrin antibody

<400> 90
caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg ctttgtactt tgactactgg gggcaaggga ccacggtcac cgtctcctca 360

<210> 91
<211> 360
<212> DNA
<213> Artificial

<220>
<223> VH gene of humanized anti-human alpha9 integrin antibody

<400> 91
caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg cttggtactt tgactactgg gggcaaggga ccacggtcac cgtctcctca 360

<210> 92
<211> 360
<212> DNA
<213> Artificial

<220>
<223> VH gene of humanized anti-human alpha9 integrin antibody

<400> 92
caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggt cttggtactt tgactactgg gggcaaggga ccacggtcac cgtctcctca 360

<210> 93
<211> 360
<212> DNA
<213> Artificial

<220>
<223> VH gene of humanized anti-human alpha9 integrin antibody

<400> 93

caggtgcagc tggtgcagtc tggggctgag gtgaagaagc ctgggtcctc cgtgaagatg 60

tcctgcaagg cttctggata caccctcacc acctactggt tgcactgggt gaaacagaga 120

cctggacaag ggcttgagtg gattggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggt gggaatactt tgactactgg gggcaaggga ccacggtcac cgtctcctca 360

<210> 94
<211> 360
<212> DNA
<213> Artificial

<220>
<223> VH gene of humanized anti-human alpha9 integrin antibody

<400> 94
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg atttctactt tgactactgg gggcaaggga ccacggtcac cgtctcctca 360

<210> 95
<211> 360
<212> DNA
<213> Artificial

<220>
<223> VH gene of humanized anti-human alpha9 integrin antibody

<400> 95
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg ctttgtactt tgactactgg gggcaaggga ccacggtcac cgtctcctca 360

<210> 96
<211> 360
<212> DNA
<213> Artificial

<220>
<223> VH gene of humanized anti-human alpha9 integrin antibody

<400> 96
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg cttggtactt tgactactgg gggcaaggga ccacggtcac cgtctcctca     360

<210>  97
<211>  360
<212>  DNA
<213>  Artificial

<220>
<223>  VH gene of humanized anti-human alpha9 integrin antibody

<400>  97
caggtgcagc tggtgcagtc tggggctgag gtgaagaagc ctgggtcctc cgtgaaggtc      60

tcctgcaagg cttctggata caccctcacc acctactgga tgcactgggt gcgacaggcc     120

cctggacaag ggcttgagtg gatgggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggt cttggtactt tgactactgg gggcaaggga ccacggtcac cgtctcctca     360

<210>  98
<211>  360
<212>  DNA
<213>  Artificial

<220>
<223>  VH gene of humanized anti-human alpha9 integrin antibody

<400>  98
caggtgcagc tggtgcagtc tggggctgag gtgaagaagc ctgggtcctc cgtgaaggtc      60

tcctgcaagg cttctggata caccctcacc acctactgga tgcactgggt gcgacaggcc     120

cctggacaag ggcttgagtg gatgggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggt gggaatactt tgactactgg gggcaaggga ccacggtcac cgtctcctca     360

<210>  99
<211>  360
<212>  DNA
<213>  Artificial

<220>
<223>  VH gene of humanized anti-human alpha9 integrin antibody

<400>  99
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg atttctactt tgactactgg gggcaaggga ccacggtcac cgtctcctca     360

<210>  100
<211>  360
<212>  DNA
<213>  Artificial

<220>
<223>  VH gene of humanized anti-human alpha9 integrin antibody

<400>  100
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg ctttgtactt tgactactgg gggcaaggga ccacggtcac cgtctcctca     360

<210>  101
<211>  360
<212>  DNA
<213>  Artificial

<220>
<223>  VH gene of humanized anti-human alpha9 integrin antibody

<400>  101
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggg cttggtactt tgactactgg gggcaaggga ccacggtcac cgtctcctca     360

<210>  102
<211>  360
<212>  DNA
<213>  Artificial

<220>
<223>  VH gene of humanized anti-human alpha9 integrin antibody

<400>  102
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tcaaggacag agtcacgatt accgcggaca aatccacgag cacagcctac     240

atggagctga gcagcctgag atctgaggac acggccgtgt attactgtgc gatttatggt     300

gactatgggt cttggtactt tgactactgg gggcaaggga ccacggtcac cgtctcctca     360

<210>  103
<211>  360
<212>  DNA
<213>  Artificial

<220>
<223>  VH gene of humanized anti-human alpha9 integrin antibody

<400>  103
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tcaaggacag agtcacgatt accgcggaca aatccacgag cacagcctac 240

atggagctga gcagcctgag atctgaggac acggccgtgt attactgtgc gatttatggt 300

gactatgggt gggaatactt tgactactgg gggcaaggga ccacggtcac cgtctcctca 360

<210> 104
<211> 360
<212> DNA
<213> Artificial

<220>
<223> VH gene of humanized anti-human alpha9 integrin antibody

<400> 104
caggtgcagc tggtgcagtc tggggctgag gtgaagaagc ctgggtcctc cgtgaaggtc 60

tcctgcaagg cttctggata caccctcacc acctactggt tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg atttctactt tgactactgg gggcaaggga ccacggtcac cgtctcctca 360

<210> 105
<211> 360
<212> DNA
<213> Artificial

<220>
<223> VH gene of humanized anti-human alpha9 integrin antibody

<400> 105
caggtgcagc tggtgcagtc tggggctgag gtgaagaagc ctgggtcctc cgtgaaggtc 60

tcctgcaagg cttctggata caccctcacc acctactggt tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg ctttgtactt tgactactgg gggcaaggga ccacggtcac cgtctcctca 360

<210> 106
<211> 360
<212> DNA
<213> Artificial

<220>
<223> VH gene of humanized anti-human alpha9 integrin antibody

<400> 106
caggtgcagc tggtgcagtc tggggctgag gtgaagaagc ctgggtcctc cgtgaaggtc 60

tcctgcaagg cttctggata caccctcacc acctactggt tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg cttggtactt tgactactgg gggcaaggga ccacggtcac cgtctcctca 360

<210> 107

<211> 360
<212> DNA
<213> Artificial

<220>
<223> VH gene of humanized anti-human alpha9 integrin antibody

<400> 107
caggtgcagc tggtgcagtc tggggctgag gtgaagaagc ctgggtcctc cgtgaaggtc 60

tcctgcaagg cttctggata caccctcacc acctactggt tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggt cttggtactt tgactactgg gggcaaggga ccacggtcac cgtctcctca 360

<210> 108
<211> 360
<212> DNA
<213> Artificial

<220>
<223> VH gene of humanized anti-human alpha9 integrin antibody

<400> 108
caggtgcagc tggtgcagtc tggggctgag gtgaagaagc ctgggtcctc cgtgaaggtc 60

tcctgcaagg cttctggata caccctcacc acctactggt tgcactgggt gcgacaggcc 120

cctggacaag ggcttgagtg gatgggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggt gggaatactt tgactactgg gggcaaggga ccacggtcac cgtctcctca 360

<210> 109
<211> 360
<212> DNA
<213> Artificial

<220>
<223> VH gene of humanized anti-human alpha9 integrin antibody

<400> 109
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg atttctactt tgactactgg gggcaaggga ccacggtcac cgtctcctca 360

<210> 110
<211> 360
<212> DNA
<213> Artificial

<220>
<223> VH gene of humanized anti-human alpha9 integrin antibody

<400> 110
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg ctttgtactt tgactactgg gggcaaggga ccacggtcac cgtctcctca 360

<210> 111
<211> 360
<212> DNA
<213> Artificial

<220>
<223> VH gene of humanized anti-human alpha9 integrin antibody

<400> 111
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggg cttggtactt tgactactgg gggcaaggga ccacggtcac cgtctcctca 360

<210> 112
<211> 360
<212> DNA
<213> Artificial

<220>
<223> VH gene of humanized anti-human alpha9 integrin antibody

<400> 112
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggt cttggtactt tgactactgg gggcaaggga ccacggtcac cgtctcctca 360

<210> 113
<211> 360
<212> DNA
<213> Artificial

<220>
<223> VH gene of humanized anti-human alpha9 integrin antibody

<400> 113
caggtgcagc tggtgcagtc tggggctgag gtgaagaagc ctgggtcctc cgtgaaggtc 60

tcctgcaagg cttctggata caccctcacc acctactgga tgcactgggt gcgacaggcc 120

cctggacaag ggcttgagtg gatgggatac attaatccta gctctggtta tactgaatac 180

aatcagaagt tccagggcag agtcacgatt accgcggaca aatccacgag cacagcctac 240

atggagctga gcagcctgag atctgaggac acggccgtgt attactgtgc gatttatggt 300

gactatgggt gggaatactt tgactactgg gggcaaggga ccacggtcac cgtctcctca     360

<210>  114
<211>  360
<212>  DNA
<213>  Artificial

<220>
<223>  VH gene of humanized anti-human alpha9 integrin antibody

<400>  114
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg atttctactt tgactactgg gggcaaggga ccacggtcac cgtctcctca     360

<210>  115
<211>  360
<212>  DNA
<213>  Artificial

<220>
<223>  VH gene of humanized anti-human alpha9 integrin antibody

<400>  115
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg ctttgtactt tgactactgg gggcaaggga ccacggtcac cgtctcctca     360

<210>  116
<211>  360
<212>  DNA
<213>  Artificial

<220>
<223>  VH gene of humanized anti-human alpha9 integrin antibody

<400>  116
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg cttggtactt tgactactgg gggcaaggga ccacggtcac cgtctcctca     360

<210>  117
<211>  360
<212>  DNA
<213>  Artificial

<220>

<223>  VH gene of humanized anti-human alpha9 integrin antibody

<400>  117
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggt cttggtactt tgactactgg ggcaagggga ccacggtcac cgtctcctca     360


<210>  118
<211>  360
<212>  DNA
<213>  Artificial

<220>
<223>  VH gene of humanized anti-human alpha9 integrin antibody

<400>  118
caggtgcagc tggtgcagtc tggggctgag gtgaagaagc ctgggtcctc cgtgaaggtc      60

tcctgcaagg cttctggata caccctcacc acctactgga ttcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggt gggaatactt tgactactgg ggcaagggga ccacggtcac cgtctcctca     360


<210>  119
<211>  360
<212>  DNA
<213>  Artificial

<220>
<223>  VH gene of humanized anti-human alpha9 integrin antibody

<400>  119
caggtgcagc tggtgcagtc tggggctgag gtgaagaagc ctgggtcctc cgtgaaggtc      60

tcctgcaagg cttctggata caccctcacc acctactggt tgcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg atttctactt tgactactgg ggcaagggga ccacggtcac cgtctcctca     360


<210>  120
<211>  360
<212>  DNA
<213>  Artificial

<220>
<223>  VH gene of humanized anti-human alpha9 integrin antibody

<400>  120
caggtgcagc tggtgcagtc tggggctgag gtgaagaagc ctgggtcctc cgtgaaggtc      60

tcctgcaagg cttctggata caccctcacc acctactggt tgcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac 240
atggagctga gcagcctgag atctgaggac acggccgtgt attactgtgc gatttatggt 300
gactatgggg ctttgtactt tgactactgg gggcaaggga ccacggtcac cgtctcctca 360

<210> 121
<211> 360
<212> DNA
<213> Artificial

<220>
<223> VH gene of humanized anti-human alpha9 integrin antibody

<400> 121
caggtgcagc tggtgcagtc tggggctgag gtgaagaagc ctgggtcctc cgtgaaggtc 60
tcctgcaagg cttctggata caccctcacc acctactggt tgcactgggt gcgacaggcc 120
cctggacaag ggcttgagtg gatgggatac attaatccta gctctggtta tactgaatac 180
aatcagaagt tccagggcag agtcacgatt accgcggaca aatccacgag cacagcctac 240
atggagctga gcagcctgag atctgaggac acggccgtgt attactgtgc gatttatggt 300
gactatgggg cttggtactt tgactactgg gggcaaggga ccacggtcac cgtctcctca 360

<210> 122
<211> 360
<212> DNA
<213> Artificial

<220>
<223> VH gene of humanized anti-human alpha9 integrin antibody

<400> 122
caggtgcagc tggtgcagtc tggggctgag gtgaagaagc ctgggtcctc cgtgaaggtc 60
tcctgcaagg cttctggata caccctcacc acctactggt tgcactgggt gcgacaggcc 120
cctggacaag ggcttgagtg gatgggatac attaatccta gctctggtta tactgaatac 180
aatcagaagt tccagggcag agtcacgatt accgcggaca aatccacgag cacagcctac 240
atggagctga gcagcctgag atctgaggac acggccgtgt attactgtgc gatttatggt 300
gactatgggt cttggtactt tgactactgg gggcaaggga ccacggtcac cgtctcctca 360

<210> 123
<211> 360
<212> DNA
<213> Artificial

<220>
<223> VH gene of humanized anti-human alpha9 integrin antibody

<400> 123
caggtgcagc tggtgcagtc tggggctgag gtgaagaagc ctgggtcctc cgtgaaggtc 60
tcctgcaagg cttctggata caccctcacc acctactggt tgcactgggt gcgacaggcc 120
cctggacaag ggcttgagtg gatgggatac attaatccta gctctggtta tactgaatac 180
aatcagaagt tccagggcag agtcacgatt accgcggaca aatccacgag cacagcctac 240
atggagctga gcagcctgag atctgaggac acggccgtgt attactgtgc gatttatggt 300
gactatgggt gggaatactt tgactactgg gggcaaggga ccacggtcac cgtctcctca 360

<210> 124
<211> 324

<212>  DNA
<213>  Artificial

<220>
<223>  VL gene of humanized anti-human alpha9 integrin antibody

<400>  124
gacatccaga tgacccagtc tccatcctcc ctgtctgcat ctgtaggaga cagagtcacc      60

atcacttgca aggccagtca gagtgtgaat actgatgttg cttggtatca gcagaaacca     120

gggaaagccc ctaagctcct gatctacttt gcatccaatc actacactgg ggtcccatca     180

aggttcagtg gaagtggatc tgggacagat tttactttca ccatcagcag cctgcagcct     240

gaagatattg caacatatta ctgtcggcag gattacagct ctccgttcac gttcggcgga     300

gggaccaagg tggagatcaa acgt                                            324

<210>  125
<211>  324
<212>  DNA
<213>  Artificial

<220>
<223>  VL gene of humanized anti-human alpha9 integrin antibody

<400>  125
gacatccaga tgacccagtc tccatcctcc ctgtctgcat ctgtaggaga cagagtcacc      60

atcacttgcc aggccagtca gagtgtgaat actgatgttg cttggtatca gcagaaacca     120

gggaaagccc ctaagctcct gatctacttt gcatccaatc actacactgg ggtcccatca     180

aggttcagtg gaagtggatc tgggacagat tttactttca ccatcagcag cctgcagcct     240

gaagatattg caacatatta ctgtcggcag gattacagct ctccgttcac gttcggcgga     300

gggaccaagg tggagatcaa acgt                                            324

<210>  126
<211>  324
<212>  DNA
<213>  Artificial

<220>
<223>  VL gene of humanized anti-human alpha9 integrin antibody

<400>  126
gacatccaga tgacccagtc tccatcctcc ctgtctgcat ctgtaggaga cagagtcacc      60

atcacttgca gggccagtca gagtgtgaat actgatgttg cttggtatca gcagaaacca     120

gggaaagccc ctaagctcct gatctacttt gcatccaatc actacactgg ggtcccatca     180

aggttcagtg gaagtggatc tgggacagat tttactttca ccatcagcag cctgcagcct     240

gaagatattg caacatatta ctgtcggcag gattacagct ctccgttcac gttcggcgga     300

gggaccaagg tggagatcaa acgt                                            324

<210>  127
<211>  120
<212>  PRT
<213>  mouse

<400>  127

Gln Val Gln Leu Gln Gln Ser Gly Ala Glu Leu Ala Asn Pro Gly Ala
1               5                   10                  15

Ser Val Lys Met Ser Cys Lys Ala Ser Gly Tyr Thr Leu Thr Thr Tyr
            20                  25                  30

Trp Met His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Thr Ser Leu Thr Ser Glu Asp Ser Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Leu Thr Val Ser Ser
        115                 120

<210> 128
<211> 360
<212> DNA
<213> mouse

<400> 128
caggtccagc ttcagcagtc tggggctgaa ctggcaaatc ctggggcctc agtgaagatg 60

tcctgcaagg cttctggcta cacattaact acctactgga tgcactgggt aaaacagagg 120

cctggacagg gtctggaatg gattggatac attaatccta gctctggtta tactgaatac 180

aatcagaagt tcaaggacaa ggccacgttg actgcagaca aatcctccag cacagcctac 240

atgcaactga ccagcctgac atctgaggac tctgcagtct attactgtgc aatctatggt 300

gactatgggg atttctactt tgactactgg ggccagggca ccactctcac agtctcctca 360

<210> 129
<211> 108
<212> PRT
<213> mouse

<400> 129

Ser Ile Val Met Thr Gln Thr Pro Lys Phe Leu Leu Val Ser Ala Gly
1               5                   10                  15

Asp Arg Val Thr Met Thr Cys Lys Ala Ser Gln Ser Val Asn Thr Asp
            20                  25                  30

Val Ala Trp Phe Gln Gln Lys Pro Gly Gln Ser Pro Lys Leu Leu Ile
        35                  40                  45

Tyr Phe Ala Ser Asn His Tyr Thr Gly Val Pro Asp Arg Phe Thr Gly
    50                  55                  60

Ser Gly Tyr Gly Thr Asp Phe Thr Phe Thr Ile Ser Thr Val Gln Ala
65                  70                  75                  80

Glu Asp Leu Ala Ile Tyr Phe Cys Arg Gln Asp Tyr Ser Ser Pro Phe

85                    90                    95

Thr Phe Gly Gly Gly Thr Lys Leu Glu Ile Lys Arg
            100                 105

<210>  130
<211>  324
<212>  DNA
<213>  mouse

<400>  130
agtattgtga tgacccagac tcccaaattc ctgcttgtat cagcaggaga cagggttacc      60

atgacctgca aggccagtca gagtgtgaat actgatgttg cttggttcca acagaagcca     120

gggcagtctc ctaaactgct gatatacttt gcatccaatc actacactgg agtccctgat     180

cgcttcactg gcagtggata tgggacggat ttcactttca ccatcagcac tgtgcaggct     240

gaagacctgg caatttattt ctgtcggcag gattacagct ctccgttcac gttcggaggg     300

gggaccaagc tggaaataaa acgg                                            324

<210>  131
<211>  92
<212>  DNA
<213>  Artificial

<220>
<223>  oligo DNA depicted in Fig.3

<400>  131
cagcaagctt gccgccacca tggaatggag ctggatcttt ctcttcctcc tgtcagtaac      60

tgcaggtgtc caatcccagg tgcagctggt gc                                    92

<210>  132
<211>  96
<212>  DNA
<213>  Artificial

<220>
<223>  oligo DNA depicted in Fig.3

<400>  132
aggcttctgg atacaccctc accacctact ggatgcactg ggtgaaacag agacctggac      60

aagggcttga gtggattgga tacattaatc ctagct                                96

<210>  133
<211>  93
<212>  DNA
<213>  Artificial

<220>
<223>  oligo DNA depicted in Fig.3

<400>  133
ggacaaatcc acgagcacag cctacatgga gctgagcagc ctgagatctg aggacacggc      60

cgtgtattac tgtgcgattt atggtgacta tgg                                   93

<210>  134
<211>  93
<212>  DNA
<213>  Artificial

<220>
<223>  oligo DNA depicted in Fig.3

<400> 134
tcgcggatcc actcacctga ggagacggtg accgtggtcc cttgccccca gtagtcaaag 60

tagaaatccc catagtcacc ataaatcgca cag 93

<210> 135
<211> 95
<212> DNA
<213> Artificial

<220>
<223> oligo DNA depicted in Fig.3

<400> 135
ggtgagggtg tatccagaag ccttgcagga catcttcacg gaggacccag gcttcttcac 60

ctcagcccca gactgcacca gctgcacctg ggatt 95

<210> 136
<211> 97
<212> DNA
<213> Artificial

<220>
<223> oligo DNA depicted in Fig.3

<400> 136
gctgtgctcg tggatttgtc cgcggtaatc gtgactctgt ccttgaactt ctgattgtat 60

tcagtataac cagagctagg attaatgtat ccaatcc 97

<210> 137
<211> 40
<212> DNA
<213> Artificial

<220>
<223> oligo DNA depicted in Fig.3

<400> 137
cagcaagctt gccgccacca tggaatggag ctggatcttt 40

<210> 138
<211> 40
<212> DNA
<213> Artificial

<220>
<223> oligo DNA depicted in Fig.3

<400> 138
tcgcggatcc actcacctga ggagacggtg accgtggtcc 40

<210> 139
<211> 93
<212> DNA
<213> Artificial

<220>
<223> oligo DNA depicted in Fig.4

<400> 139
cagcaagctt gccgccacca tgaagttgcc tgttaggctg ttggtgctga tgttctggat 60

tcctgcttcc agcagtgaca tccagatgac cca 93

<210> 140
<211> 95

<212>  DNA
<213>  Artificial

<220>
<223>  oligo DNA depicted in Fig.4

<400>  140
tattcacact ctgactggcc ttgcaagtga tggtgactct gtctcctaca gatgcagaca      60

gggaggatgg agactgggtc atctggatgt cactg                                 95


<210>  141
<211>  92
<212>  DNA
<213>  Artificial

<220>
<223>  oligo DNA depicted in Fig.4

<400>  141
aggccagtca gagtgtgaat actgatgttg cttggtatca gcagaaacca gggaaagccc      60

ctaagctcct gatctacttt gcatccaatc ac                                    92


<210>  142
<211>  91
<212>  DNA
<213>  Artificial

<220>
<223>  oligo DNA depicted in Fig.4

<400>  142
tgctgatggt gaaagtaaaa tctgtcccag atccacttcc actgaacctt gatgggaccc      60

cagtgtagtg attggatgca aagtagatca g                                     91


<210>  143
<211>  88
<212>  DNA
<213>  Artificial

<220>
<223>  oligo DNA depicted in Fig.4

<400>  143
cagattttac tttcaccatc agcagcctgc agcctgaaga tattgcaaca tattactgtc      60

ggcaggatta cagctctccg ttcacgtt                                         88


<210>  144
<211>  92
<212>  DNA
<213>  Artificial

<220>
<223>  oligo DNA depicted in Fig.4

<400>  144
tcgcggatcc aactgaggaa gcaaagttta aattctactc acgtttgatc tccaccttgg      60

tccctccgcc gaacgtgaac ggagagctgt aa                                    92


<210>  145
<211>  40
<212>  DNA
<213>  Artificial

<220>
<223>  oligo DNA depicted in Fig.4

<400> 145
cagcaagctt gccgccacca tgaagttgcc tgttaggctg 40

<210> 146
<211> 40
<212> DNA
<213> Artificial

<220>
<223> oligo DNA depicted in Fig.4

<400> 146
tcgcggatcc aactgaggaa gcaaagttta aattctactc 40

<210> 147
<211> 15
<212> PRT
<213> Artificial

<220>
<223> linker

<400> 147

Gly Gly Gly Gly Ser Gly Gly Gly Gly Ser Gly Gly Gly Gly Ser
1               5                   10                  15

<210> 148
<211> 450
<212> PRT
<213> Artificial

<220>
<223> H chain of humanized anti - human alpha9 integrin antibody

<400> 148

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Leu Thr Thr Tyr
            20                  25                  30

Trp Leu His Trp Val Arg Gln Ala Pro Gly Gln Gly Leu Glu Trp Met
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro

435                 440                 445

Gly Lys
    450

<210>  149
<211>  1350
<212>  DNA
<213>  Artificial

<220>
<223>  H gene of humanized anti - human alpha9 integrin antibody

<400>  149
caggtgcagc tggtgcagtc tggggctgag gtgaagaagc ctgggtcctc cgtgaaggtc      60

tcctgcaagg cttctggata caccctcacc acctactggt tgcactgggt gcgacaggcc     120

cctggacaag ggcttgagtg gatgggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg atttctactt tgactactgg ggcaagggga ccacggtcac cgtctcctca     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacccagacc     600

tacatctgca acgtgaatca caagcccagc aacaccaagg tggacaagaa agttgagccc     660

aaatcttgtg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac     900

agcacgtacc gtgtggtcag cgtcctcacc gtcctgcacc aggactggct gaatggcaag     960

gagtacaagt gcaaggtctc caacaaagcc ctcccagccc ccatcgagaa aaccatctcc    1020

aaagccaaag ggcagccccg agaaccacag gtgtacaccc tgcccccatc ccgggatgag    1080

ctgaccaaga accaggtcag cctgacctgc ctggtcaaag gcttctatcc cagcgacatc    1140

gccgtggagt gggagagcaa tgggcagccg gagaacaact acaagaccac gcctcccgtg    1200

ctggactccg acggctcctt cttcctctac agcaagctca ccgtggacaa gagcaggtgg    1260

cagcagggga acgtcttctc atgctccgtg atgcatgagg ctctgcacaa ccactacacg    1320

cagaagagcc tctccctgtc tccgggtaaa                                     1350

<210>  150
<211>  450
<212>  PRT
<213>  Artificial

<220>
<223>  H chain of humanized anti - human alpha9 integrin antibody

<400>  150

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Leu His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Asp Phe Tyr Phe Asp Tyr Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys

305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  151
<211>  1350
<212>  DNA
<213>  Artificial

<220>
<223>  H gene of humanized anti - human alpha9 integrin antibody

<400>  151
caggtgcagc tggtgcagtc tggggctgag gtgaagaagc ctgggtcctc cgtgaagatg      60

tcctgcaagg cttctggata caccctcacc acctactggt tgcactgggt gaaacagaga     120

cctggacaag ggcttgagtg gattggatac attaatccta gctctggtta tactgaatac     180

aatcagaagt tccagggcag agtcacgatt accgcggaca aatccacgag cacagcctac     240

atggagctga gcagcctgag atctgaggac acggccgtgt attactgtgc gatttatggt     300

gactatgggg atttctactt tgactactgg ggcaagggaa ccacggtcac cgtctcctca     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacccagacc     600

tacatctgca acgtgaatca caagcccagc aacaccaagg tggacaagaa agttgagccc     660

aaatcttgtg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcccagccc ccatcgagaa aaccatctcc 1020

aaagccaaag ggcagccccg agaaccacag gtgtacaccc tgcccccatc ccgggatgag 1080

ctgaccaaga accaggtcag cctgacctgc ctggtcaaag gcttctatcc cagcgacatc 1140

gccgtggagt gggagagcaa tgggcagccg gagaacaact acaagaccac gcctcccgtg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 152
<211> 450
<212> PRT
<213> Artificial

<220>
<223> H chain of humanized anti - human alpha9 integrin antibody

<400> 152

Gln Val Gln Leu Val Gln Ser Gly Ala Glu Val Lys Lys Pro Gly Ser
1               5                   10                  15

Ser Val Lys Met Ser Cys Lys Ala Ser Gly Tyr Thr Leu Thr Thr Tyr
            20                  25                  30

Trp Ile His Trp Val Lys Gln Arg Pro Gly Gln Gly Leu Glu Trp Ile
        35                  40                  45

Gly Tyr Ile Asn Pro Ser Ser Gly Tyr Thr Glu Tyr Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Tyr Gly Asp Tyr Gly Ala Trp Tyr Phe Asp Tyr Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro

180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  153
<211>  1350
<212>  DNA
<213>  Artificial

<220>
<223> H gene of humanized anti - human alpha9 integrin antibody

<400> 153
caggtgcagc tggtgcagtc tggggctgag gtgaagaagc ctgggtcctc cgtgaagatg 60

tcctgcaagg cttctggata caccctcacc acctactgga ttcactgggt gaaacagaga 120

cctggacaag ggcttgagtg gattggatac attaatccta gctctggtta tactgaatac 180

aatcagaagt tcaaggacag agtcacgatt accgcggaca aatccacgag cacagcctac 240

atggagctga gcagcctgag atctgaggac acggccgtgt attactgtgc gatttatggt 300

gactatgggg cttggtactt tgactactgg ggcaagggga ccacggtcac cgtctcctca 360

gcctccacca agggcccatc ggtcttcccc ctggcaccct cctccaagag cacctctggg 420

ggcacagcgg ccctgggctg cctggtcaag gactacttcc ccgaaccggt gacggtgtcg 480

tggaactcag gcgccctgac cagcggcgtg cacaccttcc cggctgtcct acagtcctca 540

ggactctact ccctcagcag cgtggtgacc gtgccctcca gcagcttggg cacccagacc 600

tacatctgca acgtgaatca caagcccagc aacaccaagg tggacaagaa agttgagccc 660

aaatcttgtg acaaaactca cacatgccca ccgtgcccag cacctgaact cctgggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcccagccc ccatcgagaa aaccatctcc 1020

aaagccaaag ggcagccccg agaaccacag gtgtacaccc tgcccccatc ccgggatgag 1080

ctgaccaaga accaggtcag cctgacctgc ctggtcaaag gcttctatcc cagcgacatc 1140

gccgtggagt gggagagcaa tgggcagccg gagaacaact acaagaccac gcctcccgtg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 154
<211> 214
<212> PRT
<213> Artificial

<220>
<223> L chain of humanized anti - human alpha9 integrin antibody

<400> 154

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Asn Thr Asp
            20                  25                  30

Val Ala Trp Tyr Gln Gln Lys Pro Gly Lys Ala Pro Lys Leu Leu Ile
        35                  40                  45

Tyr Phe Ala Ser Asn His Tyr Thr Gly Val Pro Ser Arg Phe Ser Gly

    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Arg Gln Asp Tyr Ser Ser Pro Phe
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 155
<211> 642
<212> DNA
<213> Artificial

<220>
<223> L gene of humanized anti - human alpha9 integrin antibody

<400> 155
gacatccaga tgacccagtc tccatcctcc ctgtctgcat ctgtaggaga cagagtcacc 60

atcacttgca gggccagtca gagtgtgaat actgatgttg cttggtatca gcagaaacca 120

gggaaagccc ctaagctcct gatctacttt gcatccaatc actacactgg ggtcccatca 180

aggttcagtg gaagtggatc tgggacagat tttactttca ccatcagcag cctgcagcct 240

gaagatattg caacatatta ctgtcggcag gattacagct ctccgttcac gttcggcgga 300

gggaccaagg tggagatcaa acgtactgtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gt 642

<210> 156
<211> 214
<212> PRT
<213> Artificial

<220>
<223> L chain of humanized anti - human alpha9 integrin antibody

<400> 156

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Asn Thr Asp
            20                  25                  30

Val Ala Trp Tyr Gln Gln Lys Pro Gly Lys Ala Pro Lys Leu Leu Ile
        35                  40                  45

Tyr Phe Ala Ser Asn His Tyr Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Arg Gln Asp Tyr Ser Ser Pro Phe
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 157
<211> 642
<212> DNA
<213> Artificial

<220>
<223> L gene of humanized anti - human alpha9 integrin antibody

<400> 157

gacatccaga tgacccagtc tccatcctcc ctgtctgcat ctgtaggaga cagagtcacc 60
atcacttgca aggccagtca gagtgtgaat actgatgttg cttggtatca gcagaaacca 120
gggaaagccc ctaagctcct gatctacttt gcatccaatc actacactgg ggtcccatca 180
aggttcagtg gaagtggatc tgggacagat tttactttca ccatcagcag cctgcagcct 240
gaagatattg caacatatta ctgtcggcag gattacagct ctccgttcac gttcggcgga 300
gggaccaagg tggagatcaa acgtactgtg gctgcaccat ctgtcttcat cttcccgcca 360
tctgatgagc agttgaaatc tggaactgcc tctgttgtgt gcctgctgaa taacttctat 420
cccagagagg ccaaagtaca gtggaaggtg gataacgccc tccaatcggg taactcccag 480
gagagtgtca cagagcagga cagcaaggac agcacctaca gcctcagcag caccctgacg 540
ctgagcaaag cagactacga gaaacacaaa gtctacgcct gcgaagtcac ccatcagggc 600
ctgagctcgc ccgtcacaaa gagcttcaac aggggagagt gt 642

## Description

### Technical Field

The present invention relates to a humanized anti-human α9 integrin antibody. More particularly, it relates to a humanized Y9A2 antibody having an activity to bind to a human α9 integrin protein to inhibit α9 integrin-dependent cell adhesion, and improved activity and/or property as compared to mouse Y9A2 antibody. The humanized antibody is expected to be a drug for the diagnosis, prevention or treatment of autoimmune diseases such as rheumatoid arthritis, immune diseases such as allergies and graft rejections, and other various diseases involving α9 integrin in their pathogenesis.

### Background Art

Integrin, a cell surface glycoprotein, is an adhesion molecule that functions mainly as a receptor for cell adhesion to extracellular matrices (collagen, laminin and the like) and members of the immunoglobulin family (ICAM-1, VCAM-1 and the like), and mediates signal transduction from extracellular matrices. Thereby, cells receive signals from the extracellular matrices, and differentiation, proliferation, cell death and the like are induced. Integrin is a hetero-dimer consisting of the two subunits α chain and β chain; there are different α chains and β chains occurring in a wide variety of combinations, and there are 24 members of the integrin superfamily. Integrin-knockout mice are fatal or diseased irrespective of which subunit is lacked, suggesting that individual integrins are necessary for the maintenance of life. Therefore, integrin, which transmits information on ambient conditions to cells to stimulate their responses, are thought to function in all situations of biological phenomena, and to mediate a broad range of pathologic conditions.

As such, integrin is indispensable to the survival of organisms, and is thought to play roles even in diseased states; some cases have been reported in which their inhibition helps improve pathologic conditions. For example, an inhibitor of platelet-specific integrin αIIbβ3 has been approved as a therapeutic drug for PCTA restenosis known as abciximab (trade name: ReoPro; Eli Lilly). Natalizumab (trade name: Antegren; ELAN Company), an α4β1(VLA4) inhibitor, has been approved as a therapeutic drug for multiple sclerosis. The αvβ3 inhibitor Vitaxin (MEDIMMUNE Company) is under development in clinical studies for its neovascularization inhibitory action, osteoclast activation inhibitory action and the like.

Integrin α9β1 is expressed in macrophages, NKT cells, dendritic cells, and neutrophils, and reportedly plays important roles in the infiltration and adhesion of these inflammatory cells, bone resorption and the like. Recently, it has been reported that integrin α9β1 is involved in osteoclast formation, and its involvement in bone destruction has been suggested (Non-patent Document 1). Known ligands thereof include truncated osteopontin (N-terminal OPN), VCAM-1, Tenascin-C and the like. Clinically, significantly elevated levels of integrin α9β1 have been observed in the synovial tissues of patients with rheumatoid arthritis (Non-patent Document 2).

Therefore, a monoclonal antibody that binds specifically to α9 integrin protein to act to inhibit α9 integrin-dependent cell adhesion, if developed, would be useful in the diagnosis, prevention or treatment of various diseases involving α9 integrin in their pathogenesis.

Antibodies that have been reported to exhibit function inhibitory action on human α9 integrin are the mouse monoclonal antibody Y9A2 (Non-patent Document 3), and 1K11, 24I11, 21C5 and 25B6 (Patent Document 1). In vitro experimental results have shown that these antibodies are capable of suppressing human α9 integrin-dependent cell adhesion. Among those, since Y9A2 inhibits cell adhesion to both osteopontin and Tenascin-C, it is considered most promising as a candidate for an antibody drug against α9 integrin.

It should be noted, however, that Y9A2 antibody is a mouse-derived antibody prepared by immunizing a mouse with an antigen, and therefore, direct administration thereof to human is practically impossible from the aspects of safety (induction of antigenicity) and effectiveness (shortened half-life). Therefore, a modification to convert the antibody to a molecule having an amino acid sequence of human antibody while maintaining the activity of Y9A2 antibody, i.e., humanization, needs to be performed.

At present, as a production method of humanized antibody, a method based on the method including grafting of amino acid of complementarity determining region (hereinafter sometimes to be indicated as CDR) as designed by Winter et al. (non-patent document 4) is most general. It is also well known here that simultaneous grafting of not only CDR but also non-CDR amino acid involved in the structural maintenance of CDR or binding with an antigen, i_{.}e., a framework region (hereinafter sometimes to be indicated as FR), from a foreign antibody to be the donor of CDR amino acid to a human antibody to be the acceptor of CDR is important for the reproduction of the inherent activity of the donor antibody (non-patent documents 4 and 5).

However, production of a humanized antibody based on CDR grafting includes several problems. Firstly, the most general problem is that even an appropriate selection of FR amino acid necessary for reproduction of the activity of a donor antibody cannot eliminate the difficulty of obtaining a humanized antibody having affinity to an antigen and biological activity exceeding those of the donor antibody.

In recent years, a large number of chimeric antibody, humanized antibody and human antibody has been placed in the market as monoclonal pharmaceutical products. The effective dose of any of them is extremely high and is several mg per 1 kg body weight. Therefore, antibody pharmaceuticals are inevitably expensive, which in turn increases economical burden on the patients and medical costs. The major factors defining the effective dose of an antibody drug include affinity of the antibody to an antigen and the amount of the antigen present in the body. From such aspects, particularly, an improvement in the affinity of an antibody to an antigen leads to a reduction in the dose, and is an extremely useful improvement also resulting in the reduction of economical burden on the patients and medical costs.

To realize an improved affinity of an antibody to an antigen, a method including introduction of amino acid substitution into a variable region of the antibody is often adopted. However, when antibody and antigen are different, the sequence and steric structure of CDR amino acid, as well as the position of amino acid involved in antigen-antibody interactions also vary. Therefore, it is practically impossible to define the position of FR amino acid to be grafted together with CDR as being applicable to any antibody.

Another problem is that, while the whole CDR amino acid of a donor mouse antibody is generally grafted to a template human antibody in the preparation of a humanized antibody based on CDR grafting, an amino acid sequence of CDR derived from a mouse antibody, which is important for binding with an antigen, sometimes shows antigenicity against human, often causing generation of an anti-idiotype antibody.

Furthermore, a methionine residue in an antibody is sometimes subject to oxidation during preservation of an antibody solution, often causing a decrease in the affinity to antigen. To prevent the oxidation, a method of preventing contact with oxygen, a method of avoiding preservation in a solution state and the like are sometimes employed. However, these are not necessarily basic solutions.

That is, for the production of a humanized antibody, selection of an appropriate acceptor antibody and selection of CDR amino acid and FR amino acid to be substituted are indispensable for imparting an activity higher than that of a donor antibody while avoiding generation of antigenicity in human and lowered stability of the antibody. These require considerable ingenuity and trial and error.
patent document 1: WO 2006/075784
non-patent document 1: Journal of Bone and Mineral Research, 2006, 21: 1657-1665
non-patent document 2: The Journal of Clinical Investigation, 2005, 115: 1060-1067
non-patent document 3: Am. J. Respir. Cell Mol. Biol., 1996, 15: 664-672
non-patent document 4: Science, 239, 1534-1536 (1988) non-patent document 5: Proc. Natl. Acad. Sci. USA, 86, 10029-10033 (1989)

### Disclosure of the Invention

### Problems to be Solved by the Invention

An object of the present invention is to solve the above-mentioned various problems relating to humanized antibodies, and provide a humanized anti-human α9 integrin antibody having improved activity and/or property as compared to a donor mouse anti-human α9 integrin antibody (Y9A2).

### Means of Solving the Problems

Accordingly, the present invention comprises the inventions of the following (1) - (15) as medically or industrially useful substances and methods.
(1) A humanized anti-human α9 integrin antibody comprising a heavy-chain variable region consisting of the amino acid sequence shown by SEQ ID NO: 1 or the amino acid sequence shown by SEQ ID NO: 1 wherein one or several amino acids are substituted, deleted, inserted and/or added and a light-chain variable region consisting of the amino acid sequence shown by SEQ ID NO: 61 or the amino acid sequence shown by SEQ ID NO: 61 wherein one or several amino acids are substituted, deleted, inserted and/or added.
(2) The humanized anti-human α9 integrin antibody described in the above-mentioned (1), wherein the aforementioned heavy chain variable region consists of the amino acid sequence shown by SEQ ID NO: 1 or SEQ ID NO: 31 having at least one substitution selected from the group consisting of 1) substitution of the 34th methionine residue by leucine or isoleucine in SEQ ID NO: 1 or SEQ ID NO: 31;
2) substitution of the 65th lysine residue and the 66th aspartic acid residue by glutamine and glycine, respectively, in SEO ID NO: 1 or SEQ ID NO: 31;
3) substitution of the 104th aspartic acid residue and the 105th phenylalanine residue by alanine and leucine, alanine and tryptophan, tryptophan and glutamic acid, or serine and tryptophan, respectively, in SEQ ID NO: 1 or SEQ ID NO: 31, and
   the aforementioned light chain variable region consists of the amino acid sequence shown by SEQ ID NO: 61 or the amino acid sequence shown by SEQ ID NO: 61, having
4) substitution of the 24th lysine residue by glutamine or arginine in SEQ ID NO: 61.
(3) The humanized anti-human α9 integrin antibody described in the above-mentioned (2), wherein the aforementioned heavy chain variable region consists of the amino acid sequence shown by SEQ ID NO: 1 or SEQ ID NO: 31, having 2) substitution of the 65th lysine residue and the 66th aspartic acid residue by glutamine and glycine, respectively, in SEQ ID NO: 1 or SEQ ID NO: 31, and
   the aforementioned light chain variable region consists of the amino acid sequence shown by SEQ ID NO: 61, having 4) substitution of the 24th lysine residue by glutamine or arginine in SEQ ID NO: 61.
(4) The humanized anti-human α9 integrin antibody described in the above-mentioned (1), wherein the aforementioned heavy chain variable region consists of the amino acid sequence shown by any of SEQ ID NO: 1 - 60 and the aforementioned light chain variable region consists of the amino acid sequence shown by any of SEQ ID NO: 61 - 63.
(5) The humanized anti-human α9 integrin antibody described in any of (1) to (4) above, wherein the heavy-chain constant region of the antibody is human Igγ1.
(6) The humanized anti-human α9 integrin antibody described in any of (1) to (4) above, wherein the light-chain constant region of the antibody is human Igκ.
(7) The humanized anti-human α9 integrin antibody described in any of (1) to (4) above, wherein the heavy-chain constant region of the antibody is human Igγ1 and the light-chain constant region of the antibody is human Igκ.
(8) A polynucleotide comprising a sequence that encodes the heavy-chain variable region of the humanized anti-human α9 integrin antibody described in any of (1) to (7) above.
(9) A polynucleotide comprising a sequence that encodes the light-chain variable region of the humanized anti-human α9 integrin antibody described in any of (1) to (7) above.
(10) An expression vector comprising the polynucleotide described in (8) and/or (9) above.
(11) A host cell incorporating the expression vector described in (10) above.
(12) A method of producing a humanized anti-human α9 integrin antibody, comprising a step of culturing the host cell described in (11) above to allow the cell to express the humanized anti-human α9 integrin antibody.
(13) A therapeutic drug for rheumatoid arthritis, comprising the humanized anti-human α9 integrin antibody described in any of (1) to (7) above.
(14) A method of preventing or treating rheumatoid arthritis, comprising a step of administering a therapeutically effective amount of the humanized anti-human α9 integrin antibody described in any of (1) to (7) above.
(15) A use of the humanized anti-human α9 integrin antibody described in any of (1) to (7) above in the manufacture of a pharmaceutical for preventing or treating rheumatoid arthritis.

### Effect of the Invention

According to the present invention, a humanized anti-human α9 integrin antibody having an improved activity and/or property as compared to a donor mouse anti-human α9 integrin antibody is provided. The humanized anti-human α9 integrin antibody of the present invention has a strong anti-inflammatory action and a strong bone destruction suppressive action by blocking interactions between human α9 integrin and plural ligands thereof, and is useful for the prophylaxis or treatment of various diseases involving human α9 integrin in the pathogenesis.

### Brief Description of the Drawings

Fig. 1 shows the amino acid sequence of the VH region of a mouse Y9A2 antibody.
Fig. 2 shows the amino acid sequence of the VL region of a mouse Y9A2 antibody.
Fig. 3 shows the constitution of oligoDNA for producing a gene encoding RY9A2VHv5, which is one example of VH of a humanized Y9A2 antibody.
Fig. 4 shows the constitution of oligoDNA for producing a gene encoding RY9A2VLv01, which is one example of VL of a humanized Y9A2 antibody.
Fig. 5 shows the results of Cell ELISA of chimeric Y9A2 antibody and RY9A2v501, which is an example of humanized Y9A2 antibody.
Fig. 6 shows the results of Cell ELISA of chimeric Y9A2 antibody and RY9A2v801, which is an example of humanized Y9A2 antibody.
Fig. 7 shows the results of Cell ELISA of chimeric Y9A2 antibody, RY9A2v11011 antibody and RY9A2v11012 antibody, which are humanized antibodies wherein a part of the amino acid residues of CDR of RY9A2v501 antibody is substituted by those derived from human.
Fig. 8 shows the results of Cell ELISA of chimeric Y9A2 antibody, RY9A2v5(M34L)01 antibody wherein a methionine residue in CDR of VH of RY9A2v501 antibody is substituted by leucine and RY9A2v5(M34I)01 antibody wherein the methionine residue is substituted by isoleucine.
Fig. 9 shows the results of Cell ELISA of chimeric Y9A2 antibody, RY9A2v501. antibody, RY9A2v5(M34L)01 antibody and RY9A2v5(M34I)01 antibody, each treated with hydrogen peroxide, (marked with (+) in the legend in the Figure), and those antibodies without a hydrogen peroxide treatment (marked with (-) in the legend in the Figure).
Fig. 10 shows the results of Cell ELISA of Y9A2 antibody Fab expressed by Escherichia coli and 3 kinds of modified Fabs incorporating substitution of 2 amino acids in CDR3 of VH thereof.
Fig. 11 shows the results of Cell ELISA of Y9A2 antibody Fab expressed by Escherichia coli and one kind of modified Fab incorporating substitution of 2 amino acids in CDR3 of VH thereof.

### Best Mode for Carrying out the Invention

The present invention is described in detail in the following.
The present inventors have demonstrated considerable ingenuity and consideration for the production of a humanized antibody of mouse anti-human α9 integrin antibody Y9A2, and succeeded in producing plural kinds of humanized anti-human α9 integrin antibodies (hereinafter to be also referred to as humanized Y9A2 antibody or RY9A2) having improved activities and/or properties as compared to Y9A2.

To be specific, the present inventors first grafted CDR amino acid sequence and several FR amino acids of a heavy chain variable region (hereinafter to be also referred to as VH) and a light chain variable region (hereinafter to be also referred to as VL) of Y9A2 antibody into a template human antibody to prepare humanized anti-human α9 integrin antibodies RY9A2v501 and RY9A2v801 each having an activity equivalent to that of a mouse Y9A2 antibody. CDR was determined according to the classification by Kabat et al. (Sequences of Proteins of Immunological Interest 4th ed., Public Health Service, NIH, Washington DC, 1987). The amino acid sequences of VHs of RY9A2v501 and RY9A2v801 are SEQ ID NO: 1 and SEQ ID NO: 31, respectively. Here, VH of RY9A2v801 is VH of RY9A2v501 wherein 4 amino acid residues from FR amino acid residues derived from mouse Y9A2 antibody are substituted by the corresponding template human antibody amino acids. The amino acid sequence of VLs of the both humanized antibodies is shown by SEQ ID NO: 61 and is common to the both antibodies.

Furthermore, with the aim of producing an antibody having higher affinity to human α9 integrin than that of the original mouse Y9A2 antibody, while avoiding the risk of production of antigenicity of a humanized antibody and lowered preservation stability of the antibody, the present inventors considered substitution of amino acid sequences in the CDRs of VH and VL of the above-mentioned humanized antibodies. As a result, it has been confirmed that either of the following substitutions in CDR of VH and/or VL of the humanized antibody is preferable for the improvement of the activity and/or property of humanized Y9A2 antibody as compared to the original mouse Y9A2 antibody.
1) substitution of a methionine residue (34th amino acid residue of VH amino acid sequence) in CDR1 of VH by leucine or isoleucine;
2) substitution of a lysine residue and an aspartic acid residue (65th and 66th amino acid residues of VH amino acid sequence) in CDR2 of VH by glutamine and glycine, respectively;
3) substitution of an aspartic acid residue and a phenylalanine residue (104th and 105th amino acid residues of VH amino acid sequence) in CDR3 of VH by alanine and leucine, alanine and tryptophan, tryptophan and glutamic acid, or serine and tryptophan, respectively;
4) substitution of a lysine residue (24th amino acid residue of VL amino acid sequence) in CDR1 of VL by glutamine or arginine.

That is, in a preferable embodiment, the humanized anti-human α9 integrin is an antibody having a heavy chain variable region consisting of the amino acid sequence shown by any of SEQ ID NO: 1 - 60 and a light chain variable region consisting of the amino acid sequence shown by any of SEQ ID NO: 61 - 63.

In a preferable embodiment, when the humanized anti-human α9 integrin has the substitution of the above-mentioned 2) in the VH amino acid sequence, it has the substitution of the above-mentioned 4) in the VL amino acid sequence.

The humanized anti-human α9 integrin antibody of the present invention can easily be prepared by those skilled in the art on the basis of the sequence information on the heavy-chain variable region and light-chain variable region thereof disclosed herein, using a method commonly known in the art. Specifically, a heavy-chain variable region gene segment having a base sequence that encodes the heavy-chain variable region amino acid of the antibody of the present invention, and a light-chain variable region gene segment having a base sequence that encodes the light-chain variable region amino acid of the antibody of the present invention are prepared. Then, the variable region genes are joined to a constant region gene in an appropriate class of human antibody to prepare a humanized antibody gene. Next, this humanized antibody gene is joined to an appropriate expression vector, and introduced to a cultured cell. Finally, this cultured cell is cultured, whereby a humanized antibody can be obtained from the culture supernatant.

Each of the above-described variable region gene segments that encode the heavy-chain and light-chain variable region amino acids of the antibody of the present invention can be synthesized, for example, based on the base sequences of the heavy chain and light chain variable regions or base sequences designed based on the amino acid sequences of the heavy chain and light chain variable regions and by a gene synthesis method known in the art. As such gene synthesis method, various methods known to those of ordinary skill in the art such as the antibody gene synthesis method described in WO90/07861 and the like can be used. In addition, once a variable region gene segment of the antibody of the present invention is acquired, other antibodies of the present invention can also be acquired by introducing a mutation into a given site of the gene segment. As such mutation introduction method, various methods obvious to those skilled in the art, such as site-directed mutagenesis (Current Protocols in Molecular Biology edit. Ausubel et al. (1987) Publish. John Wiley & Sons Section 8.1-8.5) can be used.

Next, the above-described variable region gene segments and the constant region gene of the human antibody are joined to prepare a humanized antibody gene. While any subclass of constant region (e.g., γ1, γ2, γ and γ4 as heavy chains, λ and κ chain constant regions as light chains) can be chosen as the constant region of the human antibody used, human Igγ1 as the heavy-chain constant region, and human Igκ as the light-chain constant region, can be preferably used.

Subsequent to the preparation of this humanized antibody gene, introduction of the humanized antibody gene to an expression vector, introduction of the expression vector to cultured cells, cultivation of the cultured cells, purification of the antibody and the like can be performed by using various methods commonly known in the art, or with reference to the methods of preparing a humanized anti-human osteopontin antibody, described in WO2007/139164 or WO2003/027151.

As the expression vector to be joined to the humanized antibody gene thus obtained, the expression vectors described in International Patent Publication Official Gazette WO94/20632, such as AG-γ1 and AG-κ, can be used, but the expression vector is not subject to limitation, as long as it is capable of expressing the humanized antibody gene. It is preferable to utilize an expression vector already having a human Ig constant region gene such as AG-γ1 or AG-κ, because it would become an expression vector having the humanized antibody gene simply when the humanized antibody variable region gene is inserted thereto. In an expression vector, a leader sequence may be used to promote extracellular secretion and expression of an antibody. As such leader sequence, a leader sequence derived from Y9A2 antibody or a leader sequence derived from other antibody (e.g., humanized anti-osteopontin antibody described in WO2007/139164) can be used.

The above-described expression vector is introduced to cultured cells by, for example, FreeStyle 293 Expression system (Invitrogen), the calcium phosphate method and the like.

As examples of the cultured cells to which the expression vector is introduced, cultured cells such as 293 cells, CHO-DG44 cells can be used, and they may be cultured by a conventional method.

After the above-described cultivation, the antibody accumulated in the culture supernatant can be purified by various chromatographies, for example, chromatography using a Protein A column.

As a method for measuring the binding activity of the obtained humanized antibody to human α9 integrin, ELISA, FACS and the like can be used. When ELISA is used, for example, cells (e.g., SW480 cell) expressing α9 integrin are immobilized on an ELISA plate, a humanized antibody is added thereto to cause reaction, and a secondary antibody such as a human IgG antibody labeled with an enzyme such as horseradish peroxidase (HRP) is added thereto to cause reaction. The cells are washed, a color development substrate (e.g., TMB when HRP labeling) is added thereto, and the absorbance is measured, whereby the antigen binding activity can be evaluated.

Furthermore, as a method for evaluating whether the obtained humanized antibody has a function inhibitory activity against human α9 integrin, it can be confirmed by an inhibition test (described in J. Biol. Chem., 274:36328-36334, 1999) of human α9 integrin molecule-dependent cell adhesion to human osteopontin molecule. That is, RAA variant (RGD sequence is altered to RAA to suppress reaction with other integrin; hereinafter sometimes to be indicated as nOPN-RAA) of N-terminal OPN (N terminal fragment after cleavage of osteopontin by thrombin; hereinafter sometimes to be indicated as nOPN), which is one of the α9 ligands, is immobilized on a plate and subjected to blocking. After addition of various antibodies, α9 expression cells are added and incubated at 37°C for 1 hr. The cells are fixed and stained with crystal violet and methanol, and washed. The dye in the adhered cells is extracted with Triton X-100, and the absorbance at wavelength 595 nm is measured. If suppressive action is confirmed thereby, the antibody is judged to possess function inhibitory activity against α9 integrin.

In addition, as a method for evaluating whether the obtained humanized antibody has resistance to oxidation, a method of treating the antibody with aqueous hydrogen peroxide is available. To be specific, aqueous hydrogen peroxide at a finial concentration of 0.4% is added to an antibody solution, and the mixture is stood still at 25°C for 4 hr and dialyzed against PBS. Then, the activity of the antibody is compared by Cell ELISA with that of an antibody without treatment, whereby the oxidation resistance can be evaluated.

The humanized anti-human α9 integrin antibody of the present invention thus obtained after being further purified as required can be prepared as a pharmaceutical preparation according to a conventional method, and used for the treatment of autoimmune diseases such as rheumatoid arthritis and the like, immune diseases such as allergy, transplant rejection and the like, and diseases wherein α9 integrin is involved in the pathogenesis such as osteoporosis, chronic obstructive pulmonary disease, cancer and the like.

The humanized anti-human α9 integrin antibody of the present invention can be used preferably as a therapeutic agent for rheumatoid arthritis. As examples of dosage forms for these therapeutic agents, a parenteral preparation such as an injection or drip infusion can be prepared, and is preferably administered by intravenous administration, subcutaneous administration and the like. In preparing a pharmaceutical preparation, carriers and additives that match these dosage forms can be used within a pharmaceutically acceptable range.

The amount of humanized anti-human α9 integrin antibody and the like added in the above-described preparation making varies depending on the severity of symptom of patient and age, the dosage form of the preparation used or the binding titer of the antibody and the like; for example, about 0.1 mg/kg to 100 mg/kg may be used.

Moreover, the present invention also encompasses a humanized anti-human α9 integrin antibody fragment such as Fab, Fab', F(ab')2, scAb, scFv, scFv-Fc and the like, which
contains the above-mentioned heavy chain and light chain variable regions and maintains the activity thereof.

The linker for joining a heavy-chain variable region (VH) and a light-chain variable region (VL), that can be used to prepare scFv, is not subject to limitation, as long as the antibody fragment of the present invention can have the above-descried characteristics; for example, a peptide consisting of the amino acid sequence shown by GGGGSGGGGSGGGGS (SEQ ID NO:147) can be mentioned. Those skilled in the art are able to prepare a fused antibody of the humanized anti-human α9 integrin antibody or antibody fragment and another peptide or protein, and to prepare a modified antibody with a modifying agent bound thereto, on the basis of the present invention. The other peptide or protein used for the fusion is not subject to limitation, as long as it does not reduce the binding activity of the antibody; for example, human serum albumin, various tag peptides, artificial helix motif peptide, maltose-binding proteins, glutathione S transferase, various toxins, other peptides or proteins capable of promoting multimerization and the like can be mentioned. The modifying agent used for the modification is not subject to limitation, as long as it does not reduce the binding activity of the antibody; for example, polyethylene glycol, sugar chains, phospholipids, liposomes, low-molecular compounds and the like can be mentioned.

The present invention also provides a gene that encodes the antibody of the present invention or a fragment thereof, and an expression vector comprising the same. The expression vector of the present invention is not subject to limitation, as long as it is capable of expressing a gene that encodes the antibody of the present invention or a fragment thereof in various host cells of prokaryotic cells and/or eukaryotic cells, and producing these polypeptides. For example, plasmid vectors, viral vectors (e.g., adenovirus, retrovirus) and the like can be mentioned.

The expression vector of the present invention can comprise a gene that encodes the antibody of the present invention or a fragment thereof, and a promoter functionally joined to the gene. As the promoter for expressing the polypeptide of the present invention in a bacterium, when the host is a bacterium of the genus Escherichia, for example, the Trp promoter, lac promoter, recA promoter, λPL promoter, lpp promoter, tac promoter and the like can be mentioned. As the promoter for expressing the antibody of the present invention or a fragment thereof in yeast, for example, the PH05 promoter, PGK promoter, GAP promoter, and ADH promoter can be mentioned; when the host is a bacterium of the genus Bacillus, the SL01 promoter, SP02 promoter, penP promoter and the like can be mentioned. When the host is a eukaryotic cell such as a mammalian cell, β actin promoter, CAG promoter (Niwa H. et al., Gene, 108, 193-200, 1991), the SV40-derived promoter, retrovirus promoter, heat shock promoter and the like can be mentioned.

When a bacterium, particularly Escherichia coli, is used as the host cell, the expression vector of the present invention can further comprise an initiation codon, a stop codon, a terminator region and a replicable unit. When a yeast, animal cell or insect cell is used as the host, the expression vector of the present invention can comprise an initiation codon and a stop codon. In this case, an enhancer sequence, noncoding regions on the 5' side and 3' side of a gene that encodes the polypeptide of the present invention, a splicing junction, a polyadenylation site, or a replicable unit and the like may be contained. In addition, a selection marker (e.g., tetracycline resistance gene, ampicillin resistance gene, kanamycin resistance gene, neomycin resistance gene, dihydrofolate reductase gene) conventionally used according to the object may also be contained.

The present invention also provides a transformant incorporating the gene of the present invention. Such a transformant can be prepared by, for example, transforming a host cell with the expression vector of the present invention. The host cell used to prepare a transformant is not subject to limitation, as long as it matches the aforementioned expression vector, and is transformable; various cells such as natural cells or artificially established lines of cells in common use in the technical field of the present invention (e.g., bacteria (bacteria of the genus Escherichia, bacteria of the genus Bacillus), yeasts (the genus Saccharomyces, the genus Pichia and the like), animal cells or insect cells (e.g., Sf9) and the like) can be mentioned as examples. The transformation can be performed by a method known per se.

The present invention also provides a method of producing the antibody of the present invention or a fragment thereof, comprising allowing a host cell to express the gene of the present invention, i.e., using such a transformant.

In producing the antibody of the present invention or a fragment thereof, the transformant can be cultured in nutrient medium. The nutrient medium preferably contains a carbon source and an inorganic nitrogen source or organic nitrogen source required for the growth of the transformant. As examples of the carbon source, glucose, dextran, soluble starch, sucrose and the like can be mentioned; as examples of the inorganic nitrogen source or organic nitrogen source, ammonium salts, nitrates, amino acids, corn steep liquor, peptone, casein, meat extract, soybean cake, potato extract and the like can be mentioned. If desired, other nutrients (e.g., inorganic salts (e.g., calcium chloride, sodium dihydrogen phosphate, magnesium chloride), vitamins, antibiotics (e.g., tetracycline, neomycin, ampicillin, kanamycin and the like) and the like) may be contained.

Cultivation of the transformant can be performed by a method known per se. Cultivation conditions, for example, temperature, pH of the medium, and cultivation time are selected as appropriate. For example, when the host is an animal cell, an MEM medium (Science, Vol.122, p.501, 1952), DMEM medium (Virology, Vol.8, p.396, 1959), RPMI1640 medium (J. Am. Med. Assoc., Vol.199 p.519, 1967), 199 medium (Proc. Soc. Exp. Biol. Med., Vol.73, p.1, 1950) containing about 5 to 20% fetal bovine serum and the like can be used as the medium. The pH of the medium is preferably about 6 to 8, cultivation is normally performed at about 30 to 40°C for about 15 to 72 hours, and the culture may be aerated or agitated as necessary. When the host is an insect cell, for example, Grace's medium comprising fatal bovine serum (Proc. Natl. Acad. Sci. USA, Vol.82, p.8404, 1985) and the like can be mentioned, and the pH thereof is preferably about 5 to 8. Cultivation is normally performed at about 20 to 40°C for 15 to 100 hours, and the culture may be aerated or agitated as necessary. When the host is a bacterium, an actinomyces, yeast, or a filamentous fungus, for example, a liquid medium comprising the above-described nutrient sources is appropriate. A medium having a pH of 5 to 8 is preferable. When the host is E. coli, LB medium, M9 medium (Miller et al., Exp. Mol. Genet, Cold Spring Harbor Laboratory, p.431, 1972) and the like can be mentioned as preferable media. In this case, cultivation can be normally performed at 14 to 43°C for about 3 to 24 hours, while aerating or agitating the culture as necessary. When the host is a bacterium of the genus Bacillus, cultivation can be normally performed at 30 to 40°C for about 16 to 96 hours, while aerating or agitating the culture as necessary. When the host is yeast, Burkholder's minimal medium (Bostian, Proc. Natl. Acad. Sci. USA, Vol.77, p.4505, 1980) can be mentioned as examples of the medium, and the pH is desirably 5 to 8. Cultivation is normally performed at about 20 to 35°C for about 14 to 144 hours, and the culture may be aerated or agitated as necessary.

The antibody of the present invention or a fragment thereof can be recovered, preferably isolated and purified, from a cultured transformant as described above. As examples of the method of isolation and purification, methods based on differences in solubility, such as salting-out and solvent precipitation; methods based on differences in molecular weight, such as dialysis, ultrafiltration, gel filtration, and sodium dodecyl sulfate-polyacrylamide gel electrophoresis; methods based on differences in electric charge, such as ion exchange chromatography and hydroxyl apatite chromatography; methods based on specific affinity, such as affinity chromatography; methods based on differences in hydrophobicity, such as reverse phase high performance liquid chromatography; methods based on differences in isoelectric point, such as isoelectric focusing electrophoresis; and the like can be mentioned.

The present invention is explained in detail in the following by referring to Examples, which are not to be construed as limitative.

### Examples

As for the part where commercially available kits or reagents were used, unless otherwise specified, the experiments were performed according to the attached protocol.

### <<Example 1: Production of chimeric Y9A2 antibody>>

The heavy chain variable region (VH) gene of mouse Y9A2 antibody was ligated to a human Igγ1 gene, and the light chain variable region (VL) gene was ligated to a human Igκ gene to give a mouse-human chimeric Y9A2 antibody. The procedure was as follows.

First, RNA was extracted from mouse Y9A2 antibody producing hybridoma, which was supplied by University of California at San Francisco (UCSF), with a TRIzol reagent (Invitrogen). Using the RNA as a template, cDNA was synthesized by using Random Primer and SuperScript III Reverse Transcriptase (both Invitrogen). Then, using this cDNA as a template, and a primer to a leader region and a primer to the J region designed by reference to the classification of sequences of V region and J region by Kabat et al. (Sequences of Proteins of Immunological Interest 4th ed., Public Health Service, NIH, Washington DC, 1987), a VH region was amplified with Ex Taq DNA polymerase (TAKARA BIO INC.). The primer for the above-mentioned leader region and the primer for the J region used here are added with a HindIII recognition sequence and a BamHI recognition sequence, respectively. As for VL, a VL gene segment was obtained in the same manner as with VH by using a primer conforming to the leader sequence and a primer conforming to the J region.

The thus-obtained VH and VL gene segments were digested with HindIII and BamHI (both TAKARA BIO INC.), and ligated to AG-γ1 and AG-κ (WO94/20632), respectively, which are expression vectors. AG-γ1 has a β actin promoter, a gene of human immunoglobulin constant region γ1 chain, and a neomycin resistance gene (neo) as a selection marker, and becomes a plasmid expressing a heavy chain of chimeric Y9A2 antibody by inserting a mouse Y9A2 antibody VH gene between a HindIII recognition sequence and a BamHI recognition sequence located upstream of γ1 gene. AG-κ has a β actin promoter, a gene of human immunoglobulin constant region κ chain, and a dihydrofolate reductase (dhfr) gene as a selection marker, and similarly becomes a plasmid expressing a light chain of chimeric Y9A2 antibody by inserting a mouse Y9A2 antibody VL gene between a HindIII recognition sequence and a BamHI recognition sequence located upstream of κ gene.

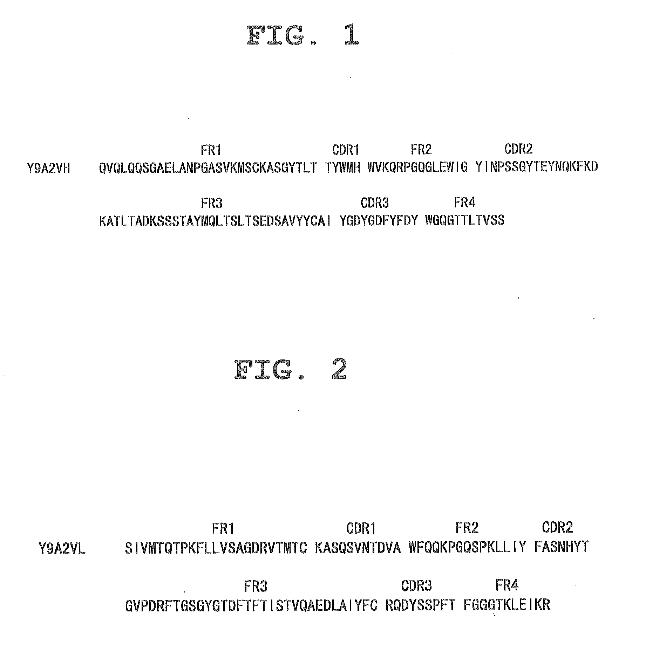

These expression plasmids were introduced into Escherichia coli according to a conventional method to give a transformed clone, from which plasmid DNA was prepared using a QIAprep Spin Miniprep Kit (QIAGEN). Using the obtained plasmid DNA as a template, and GenomeLab DTCS-Quick Start Kit and a CEQ2000 automatic sequencer (both BECKMAN COULTER), cloned VH and VL base sequences were analyzed. The base sequences of VH and VL are shown in SEQ ID NO: 128 and SEQ ID NO: 130, respectively. The amino acid sequences of VH and VL, which were determined based on the obtained sequences, are shown in Fig. 1 and Fig. 2, respectively. In addition, they are shown in SEQ ID NO: 127 and SEQ ID NO: 129, respectively.

The above-mentioned Escherichia coli clone was cultured in large amounts, and the heavy chain expression plasmid and light chain expression plasmid of chimeric Y9A2 antibody were purified using an EndoFree Plasmid Maxi Kit (QIAGEN). They were mixed, and introduced into the cell using FreeStyle 293 Expression System (Invitrogen), whereby chimeric Y9A2 antibody was transiently expressed. The concentration of the chimeric Y9A2 antibody contained in the obtained culture supernatant
was measured by sandwich ELISA using a goat-derived anti-human IgG Fc antibody (CAPPEL) and protein A-HRP (ZYMED). In this case, a dilution series of commercially available human IgG1 antibody (Biogenesis) was prepared and used as a standard sample. Then, the chimeric Y9A2 antibody in the above-mentioned culture supernatant was affinity-purified using a protein A column (GE Healthcare) to give a purified chimeric Y9A2 antibody. The concentration of the purified chimeric Y9A2 antibody was calculated wherein the concentration was 1 mg/mL when the absorbance at wavelength 280 nm was 1.4.

### «Example 2: Comparison of activities of chimeric Y9A2 antibody and mouse Y9A2 antibody»

To compare the activity of the purified chimeric Y9A2 antibody, which was prepared by the method described in the aforementioned section, with that of a mouse Y9A2 antibody (CHEMICON INTERNATIONAL), the cell adhesion inhibition test described in J. Biol. Chem., 274: 36328-36334, 1999 was performed. To be specific, a variant (nOPN-RAA) wherein RGD sequence contained in the N terminal fragment (nOPN) resulting from cleavage of osteopontin with thrombin was substituted by RAA was first immobilized and blocked, and a mouse Y9A2 antibody or a purified chimeric Y9A2 antibody was added. Successively, SW480 cell expressing a human α9 integrin molecule (hereinafter sometimes to be indicated as SW480/hα9 cell) was added, and the mixture was incubated at 37°C for 1 hr. Thereafter, the cell was fixed and stained with crystal violet and methanol and washed. The dye in the adhered cell was extracted with Triton X-100, and the absorbance at wavelength 595 nm was measured.

As a result, as shown in Table 1, it has been found that IC50 of the mouse Y9A2 antibody and that of the purified chimeric Y9A2 antibody are almost the same, and they have an activity to inhibit adhesion of SW480/hα9 cell to nOPN-RAA. The IC50 is defined to be a concentration of the anti-α9 integrin antibody necessary for suppressing 50% of the level of cell adhesion that occurs without addition of the anti-α9 integrin antibody.

**Table 1**

| Result of cell adhesion inhibition test of mouse Y9A2 antibody and chimeric Y9A2 antibody | | |
|---|---|---|
| | mouse Y9A2 antibody | chimeric Y9A2 antibody |
| IC50 (µg/mL) | 0.039 | 0.038 |

### <<Example 3: Production of humanized Y9A2 antibody gene>>

The template human antibody to be grafted with a complementarity determining region (CDR) amino acid in VH and VL of the mouse Y9A2 antibody was selected from human antibody germlines having an amino acid sequence with high homology with framework region (FR) amino acid sequence in VH, VL of the mouse Y9A2 antibody. To be specific, template human VH selected was a combination of DP-88 and JH6 and template human VL selected was a combination of DPK-1 and Jκ4.

The FRs of the above-mentioned template human antibody VH and VL were grafted with the necessary amino acid sequences from VH and VL of mouse Y9A2 antibody to give a humanized antibody. To be specific, as for VH, CDR amino acid sequence and several sites of FR amino acid of the aforementioned template human antibody VH were first substituted by the corresponding amino acid sequences in VH of mouse Y9A2 antibody. The amino acid sequences of VH of two kinds of humanized Y9A2 antibodies, i.e., RY9A2VHv5 and RY9A2VHv8, were designed (SEQ ID NO: 1 and SEQ ID NO: 31, respectively), and further, the base sequences of DNAs encoding the amino acid sequences were designed (SEQ ID NO: 64 and SEQ ID NO: 94, respectively). In RY9A2VHv8, 4 of the FR amino acid residues derived from the mouse Y9A2 antibody in RY9A2VHv5 are substituted by those of the template human antibody.

As for VL, CDR amino acid sequence of the aforementioned template human antibody VL was substituted by the amino acid sequence of CDR in VL of mouse Y9A2 antibody, the amino acid sequence of RY9A2VLv01, which is VL of humanized Y9A2 antibody, was designed (SEQ ID NO: 61), and further, the base sequence of DNA encoding the amino acid sequence was designed (SEQ ID NO: 124).

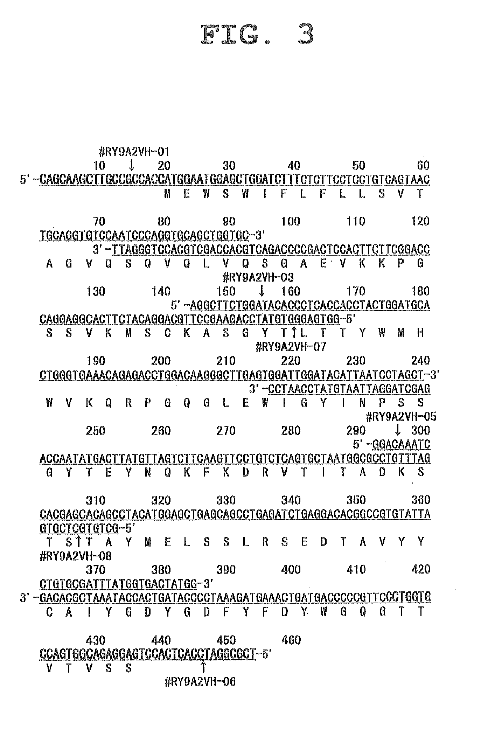

To produce DNA fragments encoding the above-mentioned RY9A2VHv5, RY9A2VHv8 and RY9A2VLv01, total synthesis was performed by PCR using oligo DNA as a material. To be specific, RY9A2VHv5 was synthesized by dividing it into 6 kinds of oligo DNAs (described in SEQ ID NO: 131 - 136) to cover the full length of VH, as respectively shown in Fig. 3 and, using them, PCR was performed by the following procedure. That is, equivalent amounts of 6 kinds of oligo DNAs were mixed. Using the mixture as a template and Pyrobest DNA polymerase (TAKARA BIO), a step of 96°C, 30 seconds, 50°C, 30 seconds and 72°C, 3 min was repeated 15 cycles. Then, using this PCR product (1
µL) as a template, oligo DNA having the sequence indicated in bold in Fig. 3 (shown by SEQ ID NOs: 137 and 138) as a primer, and Pyrobest DNA polymerase, a step of 96°C, 20 seconds and 72°C, 2 min was repeated 25 cycles to amplify full-length VH. RY9A2VHv8 was also produced generally in the same manner.

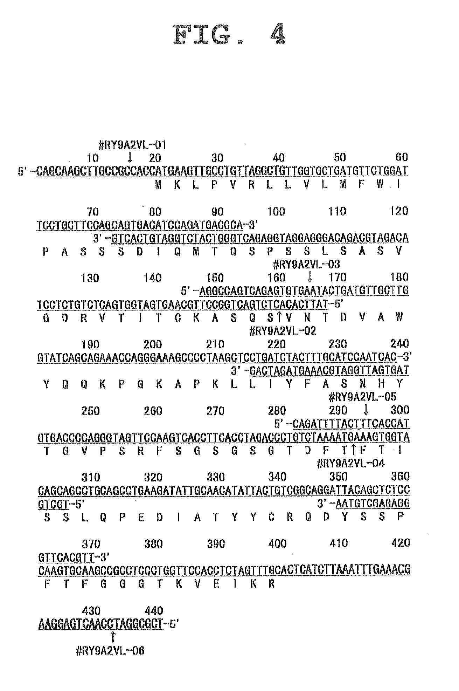

For production of RY9A2VLv01, 15 cycles of PCR were performed in the same manner as above using six oligo DNAs (shown by SEQ ID NOs: 139 - 144) shown in Fig. 4 and 25 cycles of PCR were performed in the same manner as above using the amplification product as a template, and oligo DNA having the sequence indicated in bold in Fig. 4 (shown by SEQ ID NOs: 145 and 146) as a primer, whereby the full-length VL was amplified.

In both the above-mentioned VH and VL, as a leader sequence of the antibody, a sequence same as the humanized anti-osteopontin monoclonal antibody described in WO2007/139164 was used. In addition, both ends of the obtained DNA fragment were added with a HindIII recognition sequence and a BamHI recognition sequence for cloning.

The thus-obtained DNA fragments of VH and VL were digested with restriction enzymes HindIII and BamHI, ligated with the aforementioned expression vectors AG-γ1 and AG-κ, respectively, and introduced into Escherichia coli according to a conventional method for cloning. Plasmid DNA was prepared from the obtained Escherichia coli clone using a QIAprep Spin Miniprep Kit (QIAGEN). Using the obtained plasmid DNA as a template, the base sequences of the cloned VH and VL were analyzed using a GenomeLab DTCS-Quick Start Kit and CEQ2000 automatic sequencer (both by BECKMAN COULTER), whereby clones having the designed base sequences were obtained. These clones were cultured, and expression plasmids of heavy chain and light chain were purified using an EndoFree Plasmid Maxi Kit (QIAGEN).

The purified heavy chain expression plasmid and light chain expression plasmid were mixed, and the mixture was introduced into the cell to transiently express the antibody using FreeStyle 293 Expression System. In this case, a humanized Y9A2 antibody expressed by combination of a heavy chain expression plasmid inserted with RY9A2VHv5 and a light chain expression plasmid inserted with RY9A2VLv01 was named EY9Av501 antibody, and a humanized Y9A2 antibody expressed by combination of a heavy chain expression plasmid inserted with RY9A2VHv8 and a light chain expression plasmid inserted with RY9A2VLv01 was named RY9Av801 antibody.

Measurement of the concentration of each humanized Y9A2 antibody accumulated in the culture supernatant and acquisition of a purified antibody from the culture supernatant were performed by the same method as the aforementioned chimeric Y9A2 antibody.

### <<Example 4: Confirmation of binding activity to α9 integrin molecule expression cell>>

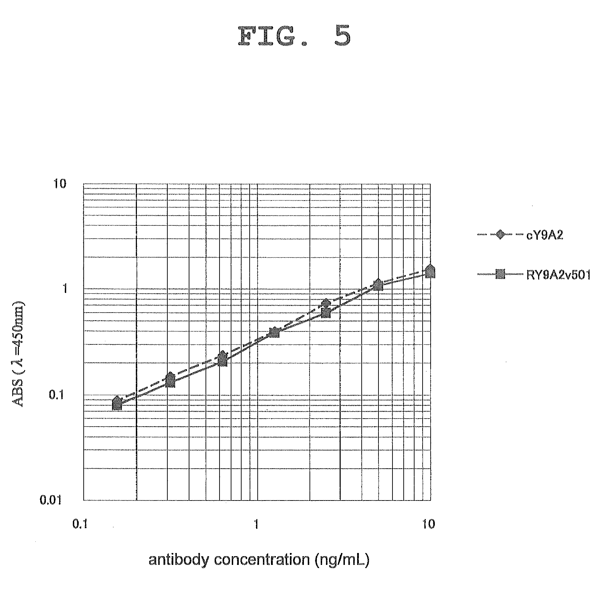

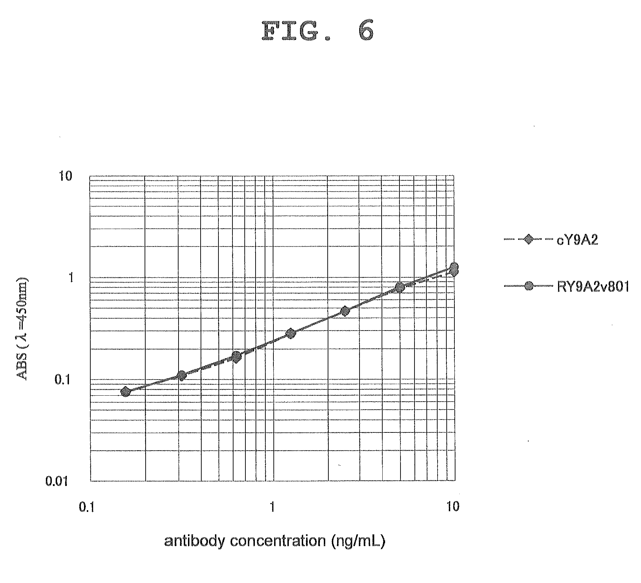

RY9A2v501 antibody and RY9A2v801 antibody expressed by the aforementioned method were compared with a chimeric Y9A2 antibody (hereinafter sometimes to be indicated as cY9A2 antibody) for the binding activity to human α9 integrin molecule according to a Cell ELISA method. To be specific, SW480 cells expressing human α9 integrin molecule are immobilized on an ELISA plate, reacted with the above-mentioned cY9A2 antibody or RY9A2v501 antibody or RY9A2v801 antibody, and reacted with HRP-labeled goat anti-human IgG(Fc) antibody (American Qualex) as a secondary antibody. TMB was added to allow color development, diluted sulfuric acid was added to quench the reaction and the absorbance at wavelength 450 nm was measured. As a result, as shown in Fig. 5 and Fig. 6, it has been confirmed that the RY9A2v501 antibody and the RY9A2v801 antibody have binding activity to human α9 integrin molecule equivalent to that of the cY9A2 antibody.

### «Example 5: Cell adhesion inhibition test of RY9A2v501 antibody, RY9A2v801 antibody»

The activity of the above-mentioned purified RY9A2v501 antibody, purified RY9A2v801 antibody and mouse Y9A2 antibody was compared by a cell adhesion inhibition test. The average values of two runs of the test are shown in Table 2.

**Table 2**

| Results of cell adhesion inhibition tests of mouse Y9A2 antibody, RY9A2v501 antibody and RY9A2v801 antibody | |
|---|---|
| antibody | IC50 (µg/mL) |
| Y9A2 | 0.070 |
| RY9A2v501 | 0.054 |
| RY9A2v801 | 0.058 |

As shown in Table 2, it has been confirmed that the above-mentioned two kinds of humanized antibodies have cell adhesion inhibitory activity equivalent to that of a mouse Y9A2.

### <<Example 6: Production of RY9A2 antibody with a part of CDR altered to human antibody sequence>>

Moreover, to avoid the risk of appearance of antigenicity of the above-mentioned two kinds of humanized antibodies in human, the possibility of substitution of amino acid in CDR of humanized antibody by human antibody amino acid was considered. As a result of the examination, substitution of the 65th lysine and the 66th aspartic acid of the heavy chain variable region (VH) of the above-mentioned humanized antibody by glutamine and glycine at the same position in a template human antibody DP-88, and substitution of the 24th lysine of the light chain variable region (VL) by glutamine or arginine derived from the germline of Vκ1 belonging to DPK1 of a template human antibody are appropriate substitutions free of a decrease in the activity of the humanized antibody.

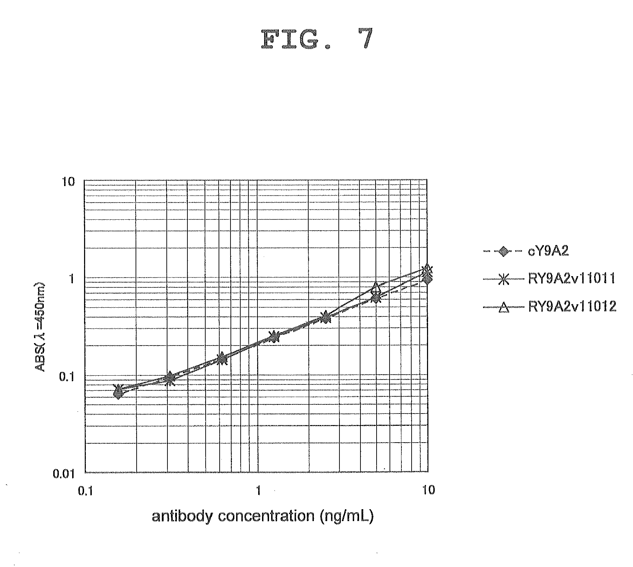

As a specific procedure, mutation was introduced by general PCR using DNA of RY9A2VHv5 and DNA of RY9A2VLv01 prepared as mentioned above as templates, and a primer containing codon changed to enable amino acid substitution. The DNA fragments of the obtained mutated VH (named RY9A2VHv11) and VL (VL containing substitution by glutamine was named RY9A2VLv011 and VL containing substitution by arginine was named RY9A2VLv012) were ligated to expression vectors AG-γ1, AG-κ in the same manner as mentioned above to give expression plasmids. Using the plasmids and FreeStyle 293 Expression System, expression was performed. The amino acid sequences of RY9A2VHv11, RY9A2VLv011 and RY9A2VLv012 are shown in SEQ ID NO: 16, SEQ ID NO: 62 and SEQ ID NO: 63, respectively, and the base sequences are shown in SEQ ID NO: 79, SEQ ID NO: 125 and SEQ ID NO: 126, respectively. The humanized antibody obtained by the expression of a combination of RY9A2VHv11 and RY9A2VLv011 was named RY9A2v11011 antibody, and the humanized antibody obtained by the expression of a combination of RY9A2VHv11 and RY9A2VLv012 was named RY9A2v11012 antibody. A comparison of the binding activity of the antibody in the expression supernatant to a human α9 integrin molecule with that of the cY9A2 antibody by Cell ELISA has revealed maintenance of binding activity equivalent to that of the cY9A2 antibody, as shown in Fig. 7.

### <<Example 7: Cell adhesion inhibition test of RY9A2v11011 antibody and RY9A2v11012 antibody>>

A purified antibody was obtained from the expression supernatant of the above-mentioned RY9A2v11011 antibody and RY9A2v11012 antibody in the same manner as above and using protein A column, and the concentration was calculated based on the absorbance at wavelength 280 nm. The activity of the obtained purified humanized antibody and mouse Y9A2 antibody was compared by a cell adhesion inhibition test and the results are shown in Table 3 (average value of two runs of test).

**Table 3**

| Results of cell adhesion inhibition tests of mouse Y9A2 antibody, RY9A2v11011 antibody and RY9A2v11012 antibody | |
|---|---|
| antibody | IC50 (µg/mL) |
| Y9A2 | 0.025 |
| RY9A2v11011 | 0.012 |
| RY9A2v11012 | 0.008 |

As shown in Table 3, the above-mentioned two kinds of humanized antibodies have been found to show an inhibitory activity improved twice or more as compared to the mouse Y9A2 antibody. To avoid the risk of appearance of antigenicity derived from the CDR amino acid sequence, amino acid was substituted. As a result, such improvement of the activity was observed, which is an effect unexpected from conventional findings.

### <<Example 8: Production of RY9A2 antibody with modified methionine residue>>

Furthermore, the present inventors examined the possibility of substitution of methionine residue in CDR of humanized antibody to avoid a decrease in the preservation stability due to the oxidation of the humanized Y9A2 antibody (RY9A2).

To be specific, the amino acid at the 34th methionine in CDR1 of VH of the above-mentioned humanized antibody was substituted. First, to select substituted amino acid free of a decrease in the binding activity to antigen, mutation introduction PCR was performed using DNA of RY9A2VHv5 as a template, whereby VH DNAs with altered codon of the above-mentioned methionine and substituted by 20 kinds of amino acids were obtained. In the same manner as in the aforementioned method, expression plasmids incorporating them were produced, which were combined with a light chain expression plasmid of RY9A2VLv01 to allow expression. The VH containing substitution by leucine was named RY9A2VHv5 (M34L) and VH containing substitution by isoleucine was named RY9A2VHv5(M34I). In addition, the humanized antibody obtained by the expression of a combination of RY9A2VHv5(M34L) and RY9A2VLv01 was named RY9A2v5(M34L)01 antibody, and the humanized antibody obtained by the expression of a combination of RY9A2VHv5(M34I) and RY9A2VLv01 was named RY9A2v5(M34I)01 antibody. The amino acid sequences of RY9A2VHv5(M34L) and RY9A2VHv5(M34I) are shown in SEQ ID NO: 11 and SEQ ID NO: 6, respectively, and the base sequences are shown in SEQ ID NO: 74 and SEQ ID NO: 69, respectively.

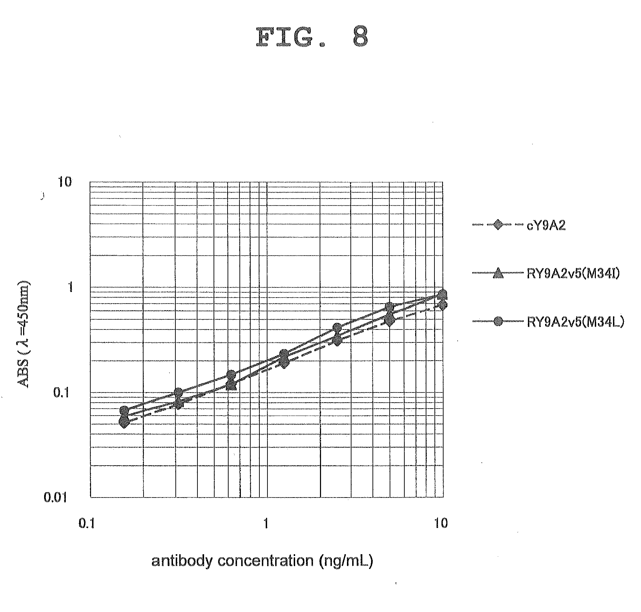

Using the supernatant obtained by the above-mentioned expression, the binding activity was evaluated based on the comparison to the cY9A2 antibody by cell ELISA. As a result, the above-mentioned two kinds of humanized antibodies were found to have antigen binding activity equivalent to that of the cY9A2 antibody, as shown in Fig. 8.

### <<Example 9: Cell adhesion inhibitory activity of RY9A2v5(M34L)01 antibody and RY9A2v5(M34I)01 antibody>>

A purified antibody was obtained from the expression supernatant of the above-mentioned RY9A2v5(M34L)01 antibody and RY9A2v5(M34I)01 antibody in the same manner as above and using protein A column, and the concentration was calculated based on the absorbance at wavelength 280 nm. The activity of the obtained purified humanized antibody and mouse Y9A2 antibody was compared by a cell adhesion inhibition test and the results are shown in Table 4 (average value of two runs of test).

**[Table 4]**

| Results of cell adhesion inhibition tests of mouse Y9A2 antibody, RY9A2v5(M34L)01 antibody and RY9A2v5(M34I)01 antibody | |
|---|---|
| antibody | IC50 (µg/mL) |
| Y9A2 | 0.049 |
| RY9A2v5(M34L)01 | 0.021 |
| RY9A2v5(M34I)01 | 0.024 |

As shown in Table 4, the above-mentioned two kinds of humanized antibodies have been found to show an about twice-improved inhibitory activity as compared to the mouse Y9A2 antibody. The improved inhibitory activity even after amino acid substitution aiming to avoid a decrease in the antibody stability was a preferable effect not predicted at first.

### <<Example 10: Confirmation of resistance to oxidation of RY9A2v5(M34L)01 antibody and RY9A2v5(M34I)01 antibody>>

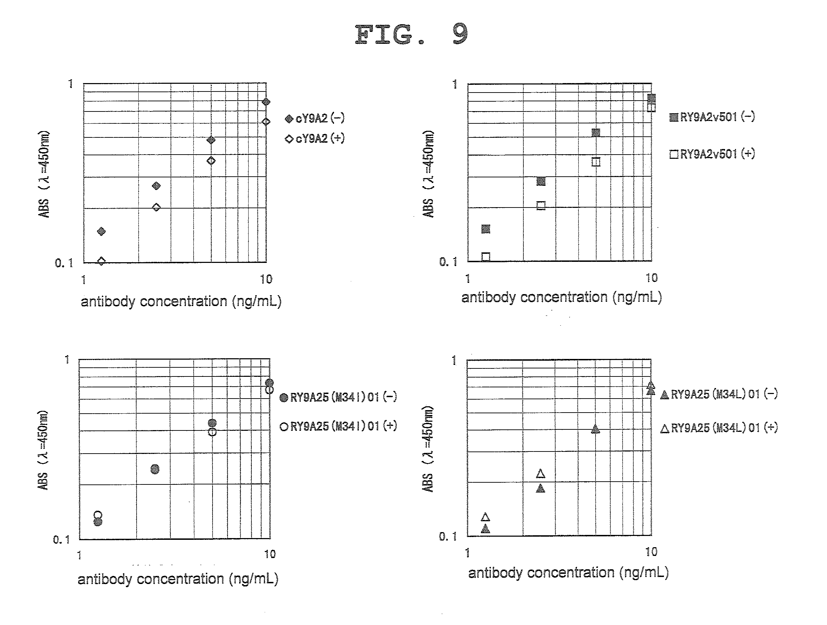

Whether RY9A2v5(M34L)01 antibody and RY9A2v5(M34I)01 antibody produced as mentioned above in fact have resistance to oxidation was examined by the following method. First, to solutions of cY9A2 antibody, RY9A2v501 antibody, RY9A2v5(M34L)01 antibody and RY9A2v5(M34I)01 antibody was added aqueous hydrogen peroxide at a final concentration of 0.4%. The mixtures were stood still at 25°C for 4 hr and dialyzed against PBS. Then, they were compared and analyzed by Cell ELISA with an antibody free of a treatment with hydrogen peroxide. As a result, as shown in Fig. 9, the cY9A2 antibody and RY9A2v501 antibody with the 34th methionine showed decreased antigen binding activity due to the hydrogen peroxide treatment, whereas the RY9A2v5(M34L)01 antibody and RY9A2v5(M341)01 antibody did not show decreased binding activity due to the hydrogen peroxide treatment.

As mentioned above, it has been found that alteration of the 34th methionine of Y9A2 antibody to leucine or isoleucine imparts resistance to oxidation while maintaining the affinity to a human α9 integrin molecule.

### <<Example 11: Production of Y9A2 antibody Fab>>

Y9A2 antibody Fab was produced as follows using Escherichia coli. First, VH and VL regions of Y9A2 antibody were amplified by PCR, and introduced into a pTrc99A modified vector described in J. Biochem. 138, 1-10 (2005) to give expression plasmid of Y9A2 antibody Fab. The pTrc99A modified vector incorporates a gene of human Igγ1 CH1 domain and a gene of human Igκ domain, and genes of VH and VL domains can be respectively introduced into the upstream thereof. In addition, a pelB signal necessary for secretion-expression is added to the upstream of VH and VL, and protein expression is regulated by trc promoter and LacI repressor. The above-mentioned Y9A2 antibody Fab expression plasmid was introduced into Escherichia coli JM83 strain, and the strain was cultured in a 2xYT medium at 30°C. 1 mM IPTG was added, and the strain was cultured overnight to allow expression of Fab in the supernatant. Fab of the Y9A2 antibody contained in several hundred mL of the culture supernatant was purified by a protein G column (GE Healthcare), and the concentration was calculated based on the absorbance at a wavelength of 280 nm.

### <<Example 12: Introduction of mutation into Y9A2 antibody Fab>>

Moreover, the present inventors have considered the possibility of substitution of amino acid residue in other CDR so as to improve the activity of humanized Y9A2 antibody.

To be specific, the present inventors took note of the 104th aspartic acid and the 105th phenylalanine from the N terminal in CDR3 of VH as amino acid residues in CDR possibly indirectly influencing binding to antigen, though not directly. Since the present inventors had sufficient experience and inventive idea, they took note of such residues, which cannot be envisaged easily based on known facts. Using a primer wherein the codon site encoding the residue was altered to NNK (N means A or T or G or C, and K means G or T) and GeneTailor Site-Directed Mutagenesis System (Invitrogen), a plasmid having a mutation introduced into the above-mentioned part of the gene of the aforementioned mouse Y9A2 antibody Fab was produced. The plasmid was introduced into Escherichia coli for cloning, and Fab was expressed individually in approximately 900 clones. The concentration of the amino acid-substituted Fab secreted in the supernatant was calculated by the following ELISA. That is, goat anti-human IgG-Fab (BETHYL) antibody was immobilized on an ELISA plate, and a culture supernatant containing Fab was added to allow reaction. Then, HRP-labeled goat anti-human Igκ antibody (American Qualex) was bound thereto, a TMB reagent was added to allow color development, dilute sulfuric acid was added to quench the reaction, and the absorbance at wavelength 450 nm was measured. As a standard sample for concentration calculation, Fab of the aforementioned purified Y9A2 antibody was used.

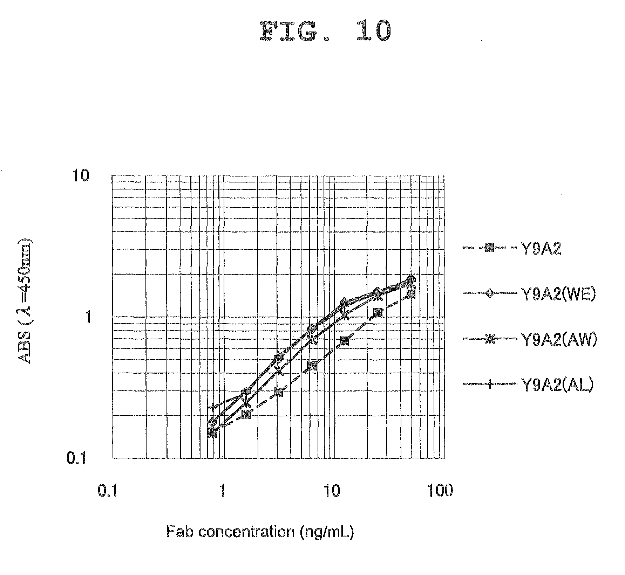

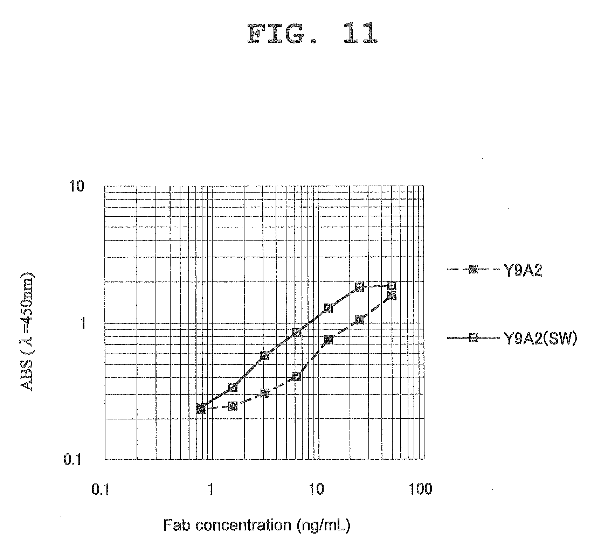

Next, for the above-mentioned Fab expression supernatant with amino acid substitution introduced thereinto, the binding activity to a human α9 integrin molecule was examined by the aforementioned Cell ELISA method. As a comparison control, simultaneous measurement of Fab of Y9A2 antibody was also performed. As a result, 4 kinds of clones wherein the 104th and 105th amino acids of the Y9A2 antibody were substituted by alanine and leucine, alanine and tryptophan, tryptophan and glutamic acid, and serine and tryptophan, respectively, were confirmed to have higher antigen binding activity than the Y9A2 antibody Fab. The results of Cell ELISA of these 4 kinds of substitutes and Y9A2 antibody Fab are shown in Fig. 10 and Fig. 11.

### <<Example 13: Production of RY9A2v501 with CDR3 of VH substituted by amino acid>>

The 4 kinds of amino acid substitutes, which showed improved binding activity of Y9A2 antibody Fab to a human α9 integrin molecule in the consideration described in the above section, were introduced into VH of RY9A2v501 antibody. For introduction of the amino acid substitution, PCR was performed in the same manner as in the aforementioned CDR amino acid substitutes and using the VH gene of RY9A2v501 as a template and a primer with different codon. VHs with substitution by alanine and leucine, alanine and tryptophan, tryptophan and glutamic acid, and serine and tryptophan were named RY9A2VHv5(AL), RY9A2VHv5(AW), RY9A2VHv5(WE) and RY9A2VHv5(SW), respectively. The amino acid sequences thereof are shown in SEQ ID NO: 2, SEQ ID NO: 3, SEQ ID NO: 5 and SEQ ID NO: 4, respectively, and the base sequences thereof are shown in SEQ ID NO: 65, SEQ ID NO: 66, SEQ ID NO: 68 and SEQ ID NO: 67, respectively.

Those VH fragments were ligated to expression vector AG-γ1 in the same manner as above to give heavy chain expression plasmids, which were combined with a light chain expression plasmid of RY9A2VLv01 to allow expression. The 4 kinds of purified antibodies (antibodies containing VHs substituted by alanine and leucine, alanine and tryptophan, tryptophan and glutamic acid, and serine and tryptophan were named RY9A2v5(AL)01, RY9A2v5(AW)01, RY9A2v5(WE)01 and RY9A2v5(SW)01, respectively) were obtained from the obtained culture supernatant using a protein A column. The antibody concentration was calculated based on the absorbance at wavelength 280 nm.

### <<Example 14: Cell adhesion inhibition test of RY9A2v501 antibody substituted by 2 amino acids>>

The 4 kinds of RY9A2 antibodies produced as mentioned above were subjected to the aforementioned cell adhesion inhibition test. The average values of two runs of the test are shown in Table 5. The numerical values in the parentheses in the column of IC50 show the results of mouse Y9A2 antibody measured simultaneously with each humanized antibody. As a result, all humanized antibodies showed improved inhibitory activity as compared to the mouse Y9A2 antibody. Particularly, it has been found that RY9A2v5(AW)01 antibody wherein the 104th and the 105th were substituted by alanine and tryptophan, respectively, and RY9A2v5(WE)01 antibody wherein they were substituted by tryptophan and glutamic acid, respectively, showed markedly improved inhibitory activity, since the IC50 concentration was not higher than 1/2 as compared to the mouse Y9A2 antibody.

**[Table 5]**

| Results of cell adhesion inhibition tests of mouse Y9A2 antibody and antibodies substituted by 2 amino acids | |
|---|---|
| antibody | IC50 (µg/mL) |
| RY9A2v5(AW)01 | 0.015 (0.037) |
| RY9A2v5(SW)01 | 0.035 (0.052) |
| RY9A2v5(AL)01 | 0.032 (0.052) |
| RY9A2v5(WE)01 | 0.022 (0.052) |

### <<Example 15: Production of RY9A2 antibody introduced with CDR amino acid substitution>>

From the above-mentioned results, it has been confirmed that the following substitutions in the CDR amino acids of VH and/or VL are effective for improving the activity and/or property of RY9A2 1) substitution of a methionine residue (34th amino acid residue of VH amino acid sequence) in CDR1 of VH by leucine or isoleucine; 2) substitution of a lysine residue and an aspartic acid residue (65th and 66th amino acid residues of VH amino acid sequence) in CDR2 of VH by glutamine and glycine, respectively; 3) substitution of an aspartic acid residue and a phenylalanine residue (104th and 105th amino acid residues of VH amino acid sequence) in CDR3 of VH by alanine and leucine, alanine and tryptophan, tryptophan and glutamic acid, or serine and tryptophan, respectively; 4) substitution of a lysine residue (24th amino acid residue of VL amino acid sequence) in CDR1 of VL by glutamine or arginine.

Based on these results, VH and VL amino acids having any of the above-mentioned substitutions introduced into CDRs of VH and/or VL of RY9A2v501 and RY9A2v801 were designed. The amino acid sequences of these VH and VL (including the aforementioned VH and VL) are shown in the following Table 6-1 to Table 6-3.

**[Table 6-1]**

| Heavy chain variable region (VH) | | |
|---|---|---|
| name | amino acid sequence | base sequence |
| RY9A2VHv5-1-1 1 (RY9A2VHv5) | SEQ ID NO: 1 | SEQ ID NO: 64 |
| RY9A2VHv5-1-1-2 (RY9A2VHv5(AL)) | SEQ ID NO: 2 | SEQ ID NO: 65 |
| RY9A2VHv5-1-1-3 (RY9A2VHv5(AW)) | SEQ ID NO: 3 | SEQ ID NO: 66 |
| RY9A2VHv5-1-1-4 (RY9A2VHv5(SW)) | SEQ ID NO: 4 | SEQ ID NO: 67 |
| RY9A2VHv5-1-1-5 (RY9A2VHv5(WE)) | SEQ ID NO: 5 | SEQ ID NO: 68 |
| RY9A2VHv5-1-2-1 (RY9A2VHv5(M34I)) | SEQ ID NO: 6 | SEQ ID NO: 69 |
| RY9A2VHv5-1-2-2 | SEQ ID NO: 7 | SEQ ID NO: 70 |
| RY9A2VHv5-1-2-3 | SEQ ID NO: 8 | SEQ ID NO: 71 |
| RY9A2VHv5-1-2-4 | SEQ ID NO: 9 | SEQ ID NO: 72 |
| RY9A2VHv5-1-2-5 | SEQ ID NO: 10 | SEQ ID NO: 73 |
| RY9A2VHv5-1-3-1 (RY9A2VHv5(M34L)) | SEQ ID NO: 11 | SEQ ID NO: 74 |
| RY9A2VHv5-1-3-2 | SEQ ID NO: 12 | SEQ ID NO: 75 |
| RY9A2VHv5-1-3-3 | SEQ ID NO: 13 | SEQ ID NO: 76 |
| RY9A2VHv5-1-3-4 | SEQ ID NO: 14 | SEQ ID NO: 77 |
| RY9A2VHv5-1-3-5 | SEQ ID NO: 15 | SEQ ID NO: 78 |
| RY9A2VHv5-2-1-1 (RY9A2VHv11) | SEQ ID NO: 16 | SEQ ID NO: 79 |
| RY9A2VHv5-2-1-2 | SEQ ID NO: 17 | SEQ ID NO: 80 |
| RY9A2VHv5-2-1-3 | SEQ ID NO: 18 | SEQ ID NO: 81 |
| RY9A2VHv5-2-1-4 | SEQ ID NO: 19 | SEQ ID NO: 82 |
| RY9A2VHv5-2-1-5 | SEQ ID NO: 20 | SEQ ID NO: 83 |
| RY9A2VHv5-2-2-1 | SEQ ID NO: 21 | SEQ ID NO: 84 |
| RY9A2VHv5-2-2-2 | SEQ ID NO: 22 | SEQ ID NO: 85 |
| RY9A2VHv5-2-2-3 | SEQ ID NO: 23 | SEQ ID NO: 86 |
| RY9A2VHv5-2-2-4 | SEQ ID NO: 24 | SEQ ID NO: 87 |
| RY9A2VHv5-2-2-5 | SEQ ID NO: 25 | SEQ ID NO: 88 |

**[Table 6-2]**

| | | |
|---|---|---|
| RY9A2VHv5-2-3-1 | SEQ ID NO: 26 | SEQ ID NO: 89 |
| RY9A2VHv5-2-3-2 | SEQ ID NO: 27 | SEQ ID NO: 90 |
| RY9A2VHv5-2-3-3 | SEQ ID NO: 28 | SEQ ID NO: 91 |
| RY9A2VHv5-2-3-4 | SEQ ID NO: 29 | SEQ ID NO: 92 |
| RY9A2VHv5-2-3-5 | SEQ ID NO: 30 | SEQ ID NO: 93 |
| RY9A2VHv8-1-1-1 (RY9A2VHv8) | SEQ ID NO: 31 | SEQ ID NO: 94 |
| RY9A2VHv8-1-1-2 | SEQ ID NO: 32 | SEQ ID NO: 95 |
| RY9A2VHv8-1-1-3 | SEQ ID NO: 33 | SEQ ID NO: 96 |
| RY9A2VHv8-1-1-4 | SEQ ID NO: 34 | SEQ ID NO: 97 |
| RY9A2VHv8-1-1-5 | SEQ ID NO: 35 | SEQ ID NO: 98 |
| RY9A2VHv8-1-2-1 | SEQ ID NO: 36 | SEQ ID NO: 99 |
| RY9A2VHv8-1-2-2 | SEQ ID NO: 37 | SEQ ID NO: 100 |
| RY9A2VHv8-1-2-3 | SEQ ID NO: 38 | SEQ ID NO: 101 |
| RY9A2VHv8-1-2-4 | SEQ ID NO: 39 | SEQ ID NO: 102 |
| RY9A2VHv8-1-2-5 | SEQ ID NO: 40 | SEQ ID NO: 103 |
| RY9A2VHv8-1-3-1 | SEQ ID NO: 41 | SEQ ID NO: 104 |
| RY9A2VHv8-1-3-2 | SEQ ID NO: 42 | SEQ ID NO: 105 |
| RY9A2VHv8-1-3-3 | SEQ ID NO: 43 | SEQ ID NO: 106 |
| RY9A2VHv8-1-3-4 | SEQ ID NO: 44 | SEQ ID NO: 107 |
| RY9A2VHv8-1-3-5 | SEQ ID NO: 45 | SEQ ID NO: 108 |
| RY9A2VHv8-2-1-1 | SEQ ID NO: 46 | SEQ ID NO: 109 |
| RY9A2VHv8-2-1-2 | SEQ ID NO: 47 | SEQ ID NO: 110 |
| RY9A2VHv8-2-1-3 | SEQ ID NO: 48 | SEQ ID NO: 111 |
| RY9A2VHv8-2-1-4 | SEQ ID NO: 49 | SEQ ID NO: 112 |
| RY9A2VHv8-2-1-5 | SEQ ID NO: 50 | SEQ ID NO: 113 |
| RY9A2VHv8-2-2-1 | SEQ ID NO: 51 | SEQ ID NO: 114 |
| RY9A2VHv8-2-2-2 | SEQ ID NO: 52 | SEQ ID NO: 115 |
| RY9A2VHv8-2-2-3 | SEQ ID NO: 53 | SEQ ID NO: 116 |
| RY9A2VHv8-2-2-4 | SEQ ID NO: 54 | SEQ ID NO: 117 |
| RY9A2VHv8-2-2-5 | SEQ ID NO: 55 | SEQ ID NO: 118 |
| RY9A2VHv8-2-3-1 | SEQ ID NO: 56 | SEQ ID NO: 119 |
| RY9A2VHv8-2-3-2 | SEQ ID NO: 57 | SEQ ID NO: 120 |
| RY9A2VHv8-2-3-3 | SEQ ID NO: 58 | SEQ ID NO: 121 |
| RY9A2VHv8-2-3-4 | SEQ ID NO: 59 | SEQ ID NO: 122 |
| RY9A2VHv8-2-3-5 | SEQ ID NO: 60 | SEQ ID NO: 123 |

Light chain variable region (VL)

**[Table 6-3]**

| name | amino acid sequence | base sequence |
|---|---|---|
| RY9A2VLv1-1 (RY9A2VLv01) | SEQ ID NO: 61 | SEQ ID NO: 124 |
| RY9A2VLv1-2 (RY9A2VLv011) | SEQ ID NO: 62 | SEQ ID NO: 125 |
| RY9A2VLv1-3 (RY9A2VLv012) | SEQ ID NO: 63 | SEQ ID NO: 126 |

DNA sequences encoding the above-mentioned VH or VL are produced by the aforementioned DNA synthesis method or mutation introduction method, and respectively ligated to expression vectors AG-γ1, AG-κ in the same manner as above to give expression plasmids. Then, the resulting plasmids encoding heavy chain and light chain are expressed using FreeStyle293 Expression System. The measurement of the concentration of each humanized Y9A2 antibody accumulated in a culture supernatant and obtainment of purified antibody from the culture supernatant are performed according to methods similar to those mentioned above.

### <<Example 16: Production of improved humanized Y9A2 antibody>>

As a result of considerable ingenuity and consideration by the present inventors, they have succeeded in producing, as a humanized Y9A2 antibody having a significantly superior activity to that of a mouse Y9A2 antibody, the following three kinds of antibodies: RY9A2v12(M34L)012 antibody, RY9A2v11(M34L)012 antibody and RY9A2v5(IAW)01 antibody.

### 1. RY9A2v12(M34L)012 antibody

The heavy chain variable region (VH) of RY9A2v12(M34L)012 antibody has the amino acid sequence shown by SEQ ID NO: 56, and the light chain variable region (VL) has the amino acid sequence shown by SEQ ID NO: 63. The base sequences of the VH and VL of the RY9A2v12(M34L)012 antibody are shown by SEQ ID NOs: 119 and 126, respectively. The VH of the RY9A2v12(M34L)012 antibody is the above-mentioned RY9A2VHv8 (SEQ ID NO: 31) wherein a methionine residue (34th amino acid residue of VH amino acid sequence) in CDR1 is substituted by leucine, as well as a lysine residue and an aspartic acid residue (65th and 66th amino acid residues of VH amino acid sequence) in CDR2 are substituted by glutamine and glycine, respectively. The VL of the RY9A2v12(M34L)012 antibody is the above-mentioned RY9A2VLv01 (SEQ ID NO: 61) wherein a lysine residue (24th amino acid residue of VL amino acid sequence) in CDR1 is substituted by arginine. The heavy chain of the RY9A2v12(M34L)012 antibody is obtained by ligating VH shown by SEQ ID NO: 56 and human Igγ1 constant region, and the amino acid sequence and base sequence thereof are shown in SEQ ID NO: 148 and SEQ ID NO: 149, respectively. The light chain of the RY9A2v12(M34L)012 antibody is obtained by ligating VL shown by SEQ ID NO: 63 and human Igκ constant region, and the amino acid sequence and base sequence thereof are shown in SEQ ID NO: 154 and SEQ ID NO: 155, respectively.

### 2. RY9A2v11(M34L)012 antibody

The VH of RY9A2v11(M34L)012 antibody has the amino acid sequence shown by SEQ ID NO: 26, and VL has the amino acid sequence shown by SEQ ID NO: 63. The base sequences of VH and VL of RY9A2v11(M34L)012 antibody are shown by SEQ ID NOs: 89 and 126, respectively. The VH of the RY9A2v11(M34L)012 antibody is the above-mentioned RY9A2VHv5(SEQ ID NO: 1) wherein a methionine residue (34th amino acid residue of VH amino acid sequence) in CDR1 is substituted by leucine, as well as a lysine residue and an aspartic acid residue (65th and 66th amino acid residues of VH amino acid sequence) in CDR2 are substituted by glutamine and glycine, respectively. The VL of the RY9A2v11(M34L)012 antibody is the above-mentioned RY9A2VLv01 (SEQ ID NO: 61) wherein a lysine residue (24th amino acid residue of VL amino acid sequence) in CDR1 is substituted by arginine. The heavy chain of the RY9A2v11(M34L)012 antibody is obtained by ligating VH shown by SEQ ID NO: 26 and human Igγ1 constant region, and the amino acid sequence and base sequence thereof are shown in SEQ ID NO: 150 and SEQ ID NO: 151, respectively. The light chain of the RY9A2v12(M34L)012 antibody is obtained by ligating VL shown by SEQ ID NO: 63 and human Igκ constant region, and the amino acid sequence and base sequence thereof are shown in SEQ ID NO: 154 and SEQ ID NO: 155, respectively.

### 3. RY9A2v5(IAW)01 antibody

The VH of RY9A2v5(IAW)01 antibody has the amino acid sequence shown by SEQ ID NO: 8, and VL has the amino acid sequence shown by SEQ ID NO: 61. The base sequences of VH and VL of RY9A2v5(IAN)01 are shown by SEQ ID NOs: 71 and 124, respectively. The VH of the RY9A2v5(IAW)01 antibody is the above-mentioned RY9A2VHv5 (SEQ ID NO: 1) wherein a methionine residue (34th amino acid residue of VH amino acid sequence) in CDR1 is substituted by isoleucine, as well as an aspartic acid residue and a phenylalanine residue (104th and 105th amino acid residues of VH amino acid sequence) in CDR3 are substituted by alanine and tryptophan, respectively. The VL of RY9A2v5(IAW)01 antibody is the same as the above-mentioned RY9A2VLv01 (SEQ ID NO: 61). The heavy chain of the RY9A2v5(IAW)01 antibody is obtained by ligating VH shown by SEQ ID NO: 8 and human Igγ1 constant region, and the amino acid sequence and base sequence thereof are shown in SEQ ID NO: 152 and SEQ ID NO: 153, respectively. The light chain of the RY9A2v12(M34L)012 antibody is obtained by ligating VL shown by SEQ ID NO: 61 and human Igκ constant region, and the amino acid sequence and base sequence thereof are shown in SEQ ID NO: 156 and SEQ ID NO: 157, respectively.

The DNA fragments of VH and VL of the above-mentioned 3 kinds of improved humanized Y9A2 antibodies were ligated with the expression vectors AG-γ1 and AG-κ to give an expression plasmid in the same manner as above. The plasmid was expressed using FreeStyle 293 Expression System, and various purified antibodies were obtained from the culture supernatant by using a protein column A as mentioned above.

### <<Example 17: Confirmation of binding activity of improved humanized Y9A2 antibody to human α9 integrin>>

The 3 kinds of humanized Y9A2 antibodies and cY9A2 antibodies produced as mentioned above were confirmed for the binding activity to human α9 integrin by CELL ELISA in the same manner as in Example 4. As a result, all of the improved humanized Y9A2 antibodies could be confirmed to have binding activity to human α9 integrin, like the chimeric Y9A2 antibody.

### <<Example 18: Cell adhesion inhibition test of improved humanized Y9A2 antibody to human nOPN>>

To compare the activity of the 3 kinds of improved humanized Y9A2 antibodies with that of a mouse Y9A2 antibody, the cell adhesion inhibition test was performed in the same manner as in Example 2.

The results are shown in Table 7. The IC50 (µg/mL) is defined to be a concentration of the anti-α9 integrin antibody necessary for suppressing 50% of the level of cell adhesion that occurs without addition of the anti-α9 integrin antibody. The average of IC50 values of the mouse Y9A2 antibody was 0.039 µg/mL. Table 7 shows specific activity of humanized Y9A2 antibody when the IC50 value of mouse Y9A2 is 1. The average values of 2 or 3 runs of the tests are shown in Table 7. It has been found that 3 kinds of improved humanized Y9A2 antibodies show not less than 4-fold improved cell adhesion inhibitory activity as compared to the mouse Y9A2 antibody.

**[Table 7]**

| Results of cell adhesion inhibition test of improved humanized Y9A2 antibody | |
|---|---|
| antibody | specific activity |
| Y9A2 | 1.0 |
| RY9A2v5(IAW)01 | 0.29 |
| RY9A2v11(M34))012 | 0.23 |
| RY9A2v12(M34L)012 | 0.23 |

### <<Example 19: Cell adhesion inhibition test of improved humanized Y9A2 antibody to human VCAM-1 and human Tenascin C>>

The 3 kinds of improved humanized Y9A2 antibodies were examined using a ligand different from nOPN-RAA used for the aforementioned cell adhesion inhibition test. To be specific, a cell adhesion inhibitory action to human VCAM-1/Ig (R&D) and a human Tenascin C-RAA variant, wherein RGD sequence of human Tenascin C was substituted by RAA, was examined in the same manner.

Average values of two runs of tests are shown in Table 8. An average values of the cell adhesion inhibitory activity IC50 values of mouse Y9A2 antibody to human VCAM-1 and human Tenascin C-RAA were 0.077 µg/mL and 0.041 µg/mL, respectively. Table 8 shows the specific activity of humanized Y9A2 antibody when the IC50 value of mouse Y9A2 antibody is 1. It has been found that the 3 kinds of improved humanized Y9A2 antibodies show an inhibitory activity at least equivalent to that of the mouse Y9A2 antibody, though subject to variation depending on the ligand used.

**[Table 8]**

| Results of cell adhesion inhibition test of improved humanized Y9A2 antibody to VCAM-1 and Tenascin C | | |
|---|---|---|
| antibody | human VCAM-1 specific activity | human Tenascin C-RAA specific activity |
| Y9A2 | 1.0 | 1.0 |
| RY9A2v5(IAW)01 | 1.0 | 0.44 |
| RY9A2v11(M34L)012 | 0.44 | 0.35 |
| RY9A2v12(M34L)012 | 0.95 | 0.26 |

### <<Example 20: Cell migration inhibition test of improved humanized Y9A2 antibody>>

The 3 kinds of improved humanized Y9A2 antibodies were examined for an inhibitory action of migration activity of SW480/hα9 cell against nOPN-RAA. The experiment was based on the cell migration inhibition test described in Molecular Biology of the cell, 12: 3214-3225, 2001, with some changes made therein. To be precise, nOPN-RAA was immobilized on the upper layer of a transwell (Millipore), set on a plate, and then F15 medium containing 10% FCS was added to the lower layer. The mouse Y9A2 antibody or humanized Y9A2 antibody was added together with SW480/hα9 cell to the upper layer, and the mixture was incubated at 37°C for 16 hr. Thereafter, the cells that migrated into the lower layer of the transwell were quantified using a QCM Chemotaxis Cell Migration 24-well Assay kit (Millipore). The migration activity inhibited by the addition of an excess amount (100 µg/mL) of the mouse Y9A2 antibody is defined as a α9-dependent migration activity (100% inhibition), and the inhibitory rate of each antibody is shown in Table 9.

A significant migration inhibitory action was found only when the mouse Y9A2 antibody was added at 50 µg/mL; however, the improved humanized Y9A2 antibody produced by the present inventors showed a significant inhibitory action at a concentration of 5 µg/mL, which is 1/10 that of the mouse Y9A2 antibody. The significant difference test was performed by Student's t-test relative to an antibody-free well. ^{*}: P<0.05, **: P<0.01.

**[Table 9]**

| Results of cell migration inhibition test of improved humanized Y9A2 antibody | | |
|---|---|---|
| antibody | antibody concentration (µg/mL) | inhibitory rate (%) |
| | 5 | 28 |
| Y9A2 | 20 | 24 |
| | 50 | 99 * |
| RY9A2v5(IAW)01 | 5 | 109 ** |
| RY9A2v11(M34L)012 | 5 | 84 * |
| RY9A2v12(M34L)012 | 5 | 86 * |

The significant cell migration inhibitory action is directly linked to a clinically effective concentration, where a clinically effective concentration becoming 1/10 leads to extended administration intervals (e.g., administration of once in two weeks becomes once in several months, etc.) in clinical practice, and a blood concentration maintained at about 5 µg/mL permits development of a subcutaneous injectable preparation. Since self injection of subcutaneous injectable preparations is now permitted all over the world, the convenience in chronic diseases is strikingly improved particularly for both patients and medical institutions. Hence, improvement of biological activity by the present invention greatly contributes not only to the therapeutic effectiveness thereof but also improvement of patient compliance.

### «Example 21: Thermal stability test of improved humanized Y9A2 antibody»

The 3 kinds of the improved humanized Y9A2 antibodies and the mouse Y9A2 antibody were incubated at 70°C for 2 hr or 10 hr, and evaluated for thermal stability using the cell adhesion inhibition test described in Example 18.

The average values of 4 experiments are shown in Table 10. With the cell adhesion inhibitory activity without a heat treatment as 100%, the activity residual rate of each antibody after a heat treatment is shown. Although the mouse Y9A2 antibody showed a decrease in the activity in 2 hours, the 3 kinds of the improved humanized Y9A2 antibodies retained 85%
or more of the activity even after incubation for 10 hr. Hence, the property of being extremely stable to heat of the improved humanized Y9A2 antibody leads to the development of a highly convenient preparation permitting preservation at room temperature, among preparations for clinical development.

**[Table 10]**

| Results of thermal stability test of improved humanized Y9A2 antibody | | |
|---|---|---|
| antibody | activity residual rate (%) in 2 hr | activity residual rate (%) in 10 hr |
| Y9A2 | 27 | 33 |
| RY9A2v5(IAW)01 | 89 | 86 |
| RY9A2v11(M34L)012 | 92 | 87 |
| RY9A2v12(M34L)012 | 91 | 89 |

### Industrial Applicability

The humanized anti-human α9 integrin antibody of the present invention has improved activity and/or property as compared to a donor mouse anti-human α9 integrin antibody, and a strong anti-inflammatory action and a strong bone destruction suppressive action by blocking interactions between human α9 integrin and plural ligands thereof, and is useful for the prophylaxis or treatment of various diseases involving human α9 integrin in the pathogenesis.
This application is based on patent application Nos.
2008-004975 filed in Japan (filing date: January 11, 2008) and 2008-282496 (filing date: October 31, 2008), the contents of which are incorporated in full herein.

## Claims

1. A humanized anti-human α9 integrin antibody comprising a heavy-chain variable region consisting of the amino acid sequence shown by SEQ ID NO: 1 or the amino acid sequence shown by SEQ ID NO: 1 wherein one or several amino acids are substituted, deleted, inserted and/or added and a light-chain variable region consisting of the amino acid sequence shown by SEQ ID NO: 61 or the amino acid sequence shown by SEQ ID NO: 61 wherein one or several amino acids are substituted, deleted, inserted and/or added.

2. The humanized anti-human α9 integrin antibody according to claim 1, wherein the heavy chain variable region consists of the amino acid sequence shown by SEQ ID NO: 1 or SEQ ID NO: 31 having at least one substitution selected from the group consisting of
1) substitution of the 34th methionine residue by leucine or isoleucine in SEQ ID NO: 1 or SEQ ID NO: 31;
2) substitution of the 65th lysine residue and the 66th aspartic acid residue by glutamine and glycine, respectively, in SEQ ID NO: 1 or SEQ ID NO: 31;
3) substitution of the 104th aspartic acid residue and the 105th phenylalanine residue by alanine and leucine, alanine and tryptophan, tryptophan and glutamic acid, or serine and tryptophan, respectively, in SEQ ID NO: 1 or SEQ ID NO: 31, and
the light chain variable region consists of the amino acid sequence shown by SEQ ID NO: 61 or the amino acid sequence shown by SEQ ID NO: 61, having
4) substitution of the 24th lysine residue by glutamine or arginine in SEQ ID NO: 61.

3. The humanized anti-human α9 integrin antibody according to claim 2, wherein the heavy chain variable region consists of the amino acid sequence shown by SEQ ID NO: 1 or SEQ ID NO: 31, having
2) substitution of the 65th lysine residue and the 66th aspartic acid residue by glutamine and glycine, respectively, in SEQ ID NO: 1 or SEQ ID NO: 31, and the light chain variable region consists of the amino acid sequence shown by SEQ ID NO: 61, having 4) substitution of the 24th lysine residue by glutamine or arginine in SEQ ID NO: 61.

4. The humanized anti-human α9 integrin antibody according to any of claims 1 to 3, wherein the heavy-chain constant region of the antibody is human Igy1.

5. The humanized anti-human α9 integrin antibody according to any of claims 1 to 3, wherein the light-chain constant region of the antibody is human Igκ.

6. The humanized anti-human α9 integrin antibody according to any of claims 1 to 3, wherein the heavy-chain constant region of the antibody is human Igγ1 and the light-chain constant region of the antibody is human Igκ.

7. A polynucleotide comprising a sequence that encodes the heavy-chain variable region of the humanized anti-human α9 integrin antibody according to any of claims 1 to 6.

8. A polynucleotide comprising a sequence that encodes the light-chain variable region of the humanized anti-human α9 integrin antibody according to any of claims 1 to 6.

9. An expression vector comprising the polynucleotide according to claim 7 and/or the polynucleotide according to claim 8.

10. A host cell incorporating the expression vector according to claim 9.

11. A method of producing a humanized anti-human α9 integrin antibody, comprising a step of culturing the host cell according to claim 10 to allow the cell to express the humanized anti-human α9 integrin antibody.

12. A therapeutic drug for rheumatoid arthritis, comprising the humanized anti-human α9 integrin antibody according to any of claims 1 to 6.

13. A method of preventing or treating rheumatoid arthritis, comprising a step of administering a therapeutically effective amount of the humanized anti-human α9 integrin antibody according to any of clams 1 to 6.

14. A use of the humanized anti-human α9 integrin antibody according to any of claims 1 to 6 in the manufacture of a pharmaceutical for preventing or treating rheumatoid arthritis.
